# FASCICULE DE BREVET EUROPEEN

(11) **EP 1 487 484 B1**
(45) Date de publication et mention de la délivrance du brevet: **11.02.2009**
(21) Numéro de dépôt: 03727589.8
(22) Date de dépôt: 14.03.2003
(51) Int. Cl.: A61K 39/385, A61K 39/29, A61K 39/21, C07K 14/18, A61P 31/00, A61P 35/00

(54) **UTILISATION DE MELANGES DE LIPOPEPTIDES POUR LA FABRICATION DE VACCINS**
VERWENDUNG VON MISCHUNGEN VON LIPOPEPTIDEN ZUR BEREITUNG VON IMPFSTOFFEN
USE OF MIXTURES OF LIPOPEPTIDES FOR VACCINE PRODUCTION

(30) Priorité: 14.03.2002 FR 0203189
(43) Date de publication de la demande: 22.12.2004
(73) Titulaire: CENTRE NATIONAL DE LA RECHERCHE SCIENTIFIQUE (CNRS), 75016 Paris (FR); UNIVERSITE DE LILLE II, 59800 Lille (FR); INSTITUT NATIONAL DE LA SANTE ET DE LA RECHERCHE MEDICALE (INSERM), 75013 Paris (FR)
(72) Inventeur: GUILLET, Jean-Gérard, F-75009 Paris (FR); BOURGAULT-VILLADA, Isabelle, F-75007 Paris (FR); DUPUIS, Marion, F-75012 Paris (FR); GRAS MASSE, Hélène, F-59710 Merignies (FR); BOUREL, Line, F-59000 Lille (FR); MELNYK, Oleg, F-59112 Annoeulin (FR); JOLY, Pascal, F-59000 Lille (FR); BONNET, Dominique, F-59000 Lille (FR); MALINGUE, Frédéric, F-59800 Lille (FR); GRAND JEAN, Cyrille, F-75013 Paris (FR); GEORGES, Bertrand, F-59221 BAUVIN (FR)
(74) Mandataire: Hirsch & Associés
(86) Numéro de dépôt international: PCT/FR2003/000821
(87) Numéro de publication internationale: WO 2003/075956

(56) Documents cités:
- WO-A-01/14408
- WO-A-01/90197
- WO-A-02/20558
- GRAS-MASSE HELENE: "Chemoselective ligation and antigen vectorization." BIOLOGICALS, vol. 29, no. 3-4, 2001, pages 183-188, XP002225275 Sept.-Dec., 2001 ISSN: 1045-1056 cité dans la demande
- LE GAL FREDERIQUE-ANNE ET AL: "Lipopeptide-based melanoma cancer vaccine induced a strong MART-27-35-cytotoxic T lymphocyte response in a preclinal study." INTERNATIONAL JOURNAL OF CANCER, vol. 98, no. 2, 2002, pages 221-227, XP002225276 ISSN: 0020-7136
- BONNET D ET AL: "Chemoselective acylation of fully deprotected hydrazino acetyl peptides. Application to the synthesis of lipopetides" JOURNAL OF ORGANIC CHEMISTRY, AMERICAN CHEMICAL SOCIETY. EASTON, US, vol. 66, no. 2, 26 janvier 2001 (2001-01-26), pages 443-449, XP002225277 ISSN: 0022-3263

## Description

La présente invention se rapporte à l'utilisation pour la fabrication d'un vaccin d'un mélange comprenant au moins des lipopeptides, lesdits lipopeptides étant constitués d'un composé peptidique ayant été lié en solution, par un lien hydrazone, à au moins un vecteur lipophile de nature non peptidique.

L'utilisation pour la fabrication de vaccins de lipopeptides a déjà été décrite : ainsi, la demande de brevet PCT/FR98/02605 décrit des micelles mixtes de lipopeptides pour l'induction d'une réponse immunitaire. Cette utilisation permet, à partir d'un produit de composition chimique déterminée, de délivrer au système immunitaire une collection diversifiée, d'antigènes peptidiques qui seront présentés simultanément par les cellules présentatrices d'antigènes (CPA) en association avec les molécules du complexe majeur d'histocompatibilité (CMH) de classe I et de classe II. Il est ainsi possible d'activer des cellules spécifiques des antigènes présentés : cellules T auxiliaires (Lymphocytes T « helper », HTL), cellules B productrices d'anticorps, ou cellules T cytotoxiques (Lymphocytes T cytotoxiques, CTL).

Pour induire dans chaque individu vacciné une réponse immunitaire polyclonale protectrice, l'augmentation de la diversité épitopique de la composition vaccinale représente une des stratégies les plus prometteuses.

Plusieurs expériences ont montré que des réponses cellulaires multiples peuvent être induites de manière assez efficace à la suite d'une immunisation avec plusieurs peptides présentant de multiples épitopes modifiés au niveau de leur position C-terminale par une N-ε-palmitoyl lysine.

Ainsi, par exemple, certains cocktails de lipopeptides comprenant de 7 à 8 constituants différents ont été utilisés pour induire des réponses de cellules T spécifiques à un virus chez les primates (Bourgault-Villada et collaborateurs, 1997, FEMS Immunol. Med. Microbiol., 19, 81-87 ; Mortara et collaborateurs, 1999, J. Virol, 73, 4447-4451 ; Mortara et collaborateurs, 2000, Virology, 278, 551-561) et chez l'homme (Gahéry-Ségard et collaborateurs, 2000, J. Virol., 74, 1694-1703 ; Pialoux et collaborateurs, 2001, AIDS, 15 1239 - 1249).

L'étude de Pialoux et collaborateurs a montré qu'il était possible d'induire des réponses HTL et CTL chez une large majorité de volontaires sains ayant reçu une dose de 600 µg à 3 mg d'un mélange de 6 lipopeptides longs de 24 à 33 résidus (100 µg à 500 µg par peptide) dans le modèle d'infection par le Virus de l'Immunodéficience Humaine (Virus de type 1, VIH-1). La tolérance et l'immunogénicité du cocktail des 6 lipopeptides utilisés dans des essais de phase clinique de type I a confirmé que de telles formulations synthétiques représentent une approche alternative prometteuse aux vaccins conventionnels et recombinants.

Les lipopeptides connus dans l'art antérieur sont assemblés par synthèse peptidique en phase solide au niveau de l'extrémité C-terminale ou N-terminale des peptides. Ces lipopeptides présentent un caractère lipophile, et de ce fait tendent à s'agréger en solution aqueuse. Par conséquent, la difficulté majeure rencontrée au cours de leur production à l'échelle industrielle est associée aux étapes de purification par RP-HPLC préparative, dont la résolution est très limitée. La séparation des produits souhaités à partir des produits secondaires peut être atteinte seulement avec une charge limitée sur la colonne. Par conséquent, pour la préparation d'un vaccin à l'échelle industrielle, et lorsqu'on considère les lipopeptides branchés en position C-terminale, une grande partie du coût du travail est consacrée à la répétition des étapes chromatographiques. Ainsi, les rendements de purification sont relativement faibles, typiquement de 10 au niveau du laboratoire, et vraisemblablement plus faible à l'échelle industrielle (10-100 mg), de 1 à 2% ou même moins lorsqu'on augmente la taille du lot de vaccin par un facteur 10.

La préparation de micelles mixtes de lipopeptides a pour but d'assurer une distribution équitable de tous les ingrédients du vaccin auprès de chaque cellule présentatrice de l'antigène, ceci afin de permettre une coopération cellulaire entre lymphocytes T-auxiliaires et T-cytotoxiques par communication physique avec la cellule présentatrice de l'antigène. Pour atteindre une coopération cellulaire efficace, les lymphocytes doivent reconnaître simultanément à la surface d'une même cellule présentatrice de l'antigène des épitopes présentés avec des complexes majeurs d'histocompatibilité de type I et de type II. Les résultats obtenus en essai clinique confirment expérimentalement qu'une réponse immune efficace peut être obtenue au cours de laquelle un épitope T-auxiliaire est identifié dans un antigène lipopeptidique et un épitope T-cytotoxique situé dans un autre lipopeptide de la même composition (Gahéry-Ségard et collaborateurs, 2000, J. Virol., 74, 1694-1703 ; Pialoux et collaborateurs, 2001, AIDS, 15 1239 - 1249).

Pour obtenir les micelles mixtes de lipopeptides selon l'art antérieur, il faut purifier et caractériser individuellement chaque lipopeptide avant de réaliser leur mélange. Pour réaliser le mélange selon l'art antérieur il faut dissoudre individuellement chaque lipopeptide dans des conditions permettant d'obtenir une solvatation complète de chaque lipopeptide ingrédient du vaccin (PCT /FR98/02605). Des études par résonance magnétique nucléaire des solutions ont montré que cet état de dissolution n'était obtenu que dans des concentrations élevées d'acide acétique dans l'eau (80%). Le procédé selon l'art antérieur impliquait par conséquent un séjour relativement prolongé des lipopeptides dans l'acide acétique à 80% dans l'eau, afin de dissoudre individuellement chaque ingrédient, préparer leur mélange, homogénéiser puis réaliser la filtration stérilisante sur filtre de porosité 0,22 µm, avant dilution puis lyophilisation. La durée de séjour dans un tel solvant est peu compatible avec certains enchaînements peptidiques ou avec certains vecteurs lipophiles sensibles aux acides.

La demanderesse s'est donc fixée comme but de fabriquer des vaccins à l'échelle industrielle dont la fabrication ne présente pas les inconvénients de celle des vaccins de l'art antérieur.

L'invention a donc pour objet l'utilisation pour la fabrication d'un vaccin d'un mélange comprenant au moins des lipopeptides, lesdits lipopeptides étant obtenus de préférence par lipidation simultanée d'un mélange homogène de composés peptidiques et étant constitués d'un composé peptidique lié par un lien hydrazone, à au moins un vecteur lipophile de nature non peptidique, et sont de formule générale (I) suivante

[( (R¹) (R²)ᵢ) D -H]j - P (I)

dans laquelle (R¹)(R²)ᵢD représente le vecteur lipophile, dans lequel:
- i représente 0 ou 1,
- dans le cas où i est égal à 0, D représente une liaison,
- dans le cas où i est égal à 1, D représente un hétérocycle saturé; insaturé ou aromatique, mono- ou polycyclique,
- R¹ et R², qui peuvent être identiques ou différents, représentent chacun un groupement de formule L-f-E-f' où L représente un résidu d'un lipide, E représente un bras espaceur, et f et f' représentent des fonctions liant, respectivement, L à E et E à D, et
- P représente le composé peptidique,
- H représente le lien hydrazone formé par la ligation en solution entre une fonction aldéhyde portée par le vecteur lipophile et une fonction dérivé d'hydrazine portée par le composé peptidique,
- j représente 1 à 3.

Lorsque j représente 2 ou 3, le mélange de lipopeptides doit former une solution ou suspension homogène en milieu aqueux et présenter un caractère immunogène souhaité.

Dans un mode de réalisation préféré, l'utilisation d'au moins un mélange de lipopeptides selon la formule (I) est avantageusement tel que i est égal à 0, j est égal à 1 et en ce que le groupement L (dans la formule L-f-E-f' définie en rapport avec R¹ et R²) représente par exemple un stérol, un dérivé de stérol (tel qu'un dérivé du cholestérol) ou une chaîne carbonée saturée ou insaturée, linéaire ou ramifiée, comprenant entre 4 et 30 atomes de carbone. Une telle chaîne carbonée constitue la partie lipophile d'un acide gras, tel que l'acide palmitique (lorsque L répond à la formule CH₃- (CH₂)₁₄-) ou l'acide oléique (lorsque L répond à la formule CH₃- (CH₂)₇-CH=CH-(CH₂)₇-).

Dans un mode de réalisation très préféré selon l'invention, le dérivé de stérol est un dérivé du cholestérol et la chaîne carbonée répond à la formule CH₃-(CH₂)₁₄-.

Par ailleurs, E représente avantageusement une chaîne carbonée linéaire, ramifiée ou cyclique, saturée ou insaturée, comportant de 1 à 18 atomes de carbone et, éventuellement, 1 à 16 hétéroatomes (par exemple de l'azote ou de l'oxygène) et/ou 1 à 7 groupements sélectionnés parmi les groupements carbonyles, les hétérocycles, les hétéroaryles, les carbocycles et les aryles. E peut éventuellement être substitué par 1 à 8 groupements hydroxyles ou amino et/ou 1 à 16 atomes d'halogène (tels que le chlore ou le fluor).

De même, f représente avantageusement une liaison -CO-NH- ou -CO-O- et f' représente une liaison-NH-CO- ou -O-CO-.

Au sens de la présente invention, on entend par :
- « carbocycle » (aussi appelé « cycloalkyle ») un cycle carboné mono- ou polycyclique, comprenant entre 3 et 8 atomes de carbone, tel que le cyclopentyle ou le cyclohexyle ;
- « aryle » un cycle carboné aromatique, mono- ou polycyclique, comprenant entre 5 et 14 atomes de carbone, tel que le phényle, le naphtyle ou le crésyle ;
- « hétérocycle » un carbocycle comprenant un ou plusieurs hétéroatomes choisis parmi l'azote, l'oxygène et le soufre, tel que la pipérazine ;
- « hétéroaryle » un aryle comprenant un ou plusieurs hétéroatomes choisis parmi l'azote, l'oxygène et le soufre, tel que la pyridine, la pyrimidine ou la pyrazine.

Des exemples de vecteurs lipophiles préférés conformes à l'invention répondent aux formules (II) et (III) ci-après : 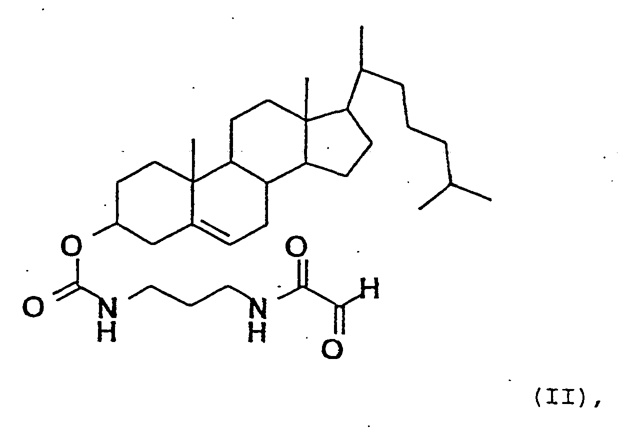 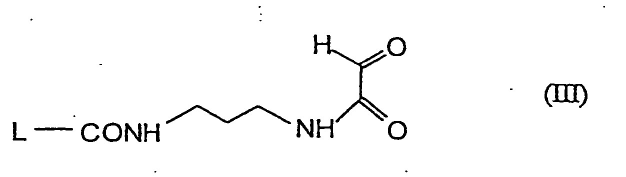 dans laquelle L est tel que défini précédemment et représente, par exemple une chaîne carbonée de formule CH₃-(CH₂)₁₄- (c' est-à-dire la partie lipophile de l'acide palmitique).

Le composé peptidique P dans la formule (I) peut être sélectionné dans le groupe constitué par un ou plusieurs antigènes peptidiques et un ou plusieurs glycomimétiques dendrimériques de nature peptidique.

Au sens de la présente invention, on entend par « un ou plusieurs » antigènes peptidiques ou glycomimétiques dendrimériques, le fait que le composé peptidique P puisse être constitué d'un seul ou de plusieurs antigènes peptidiques ou glycomimétiques dendrimériques liés entre eux, sans que l'activité du mélange de lipopeptides n'en soit modifiée.

L'antigène peptidique est avantageusement choisi parmi le groupe constitué par les peptides et les dérivés de peptides comprenant les glycopeptides et les pseudopeptides.

Au sens de la présente invention, on entend par « peptide » tout enchaînement de plusieurs acides aminés, quels que soient leur nature et leur nombre ; le terme « peptide » désigne donc aussi bien des oligopeptides (dipeptides ou tripeptides) que des polypeptides ou des protéines. On entend par « glycopeptides » des peptides associés, par liaison covalente, avec des glucides, qu'il s'agisse de monosaccharides (tels que des oses neutres) ou de polysaccharides. Par « pseudopeptides », on entend des peptides dont une ou plusieurs liaisons peptidiques (-CO-NH-) ont été remplacées par des liaisons non peptidiques (telles que les liaisons -CO-NH-NH-, -CH₂-NH- ou -CO-NH-O-).

Au sens de la présente invention, on entend par « glycomimétiques dendrimériques de nature peptidique », des glycomimétiques dont la structure est à base d'acides aminés ou de leurs dérivés et se présente sous une forme hautement ramifiée, de type arborescente.

Le glycomimétique dendrimérique de nature peptidique répond à la formule générale (IV) ci-après :

(B²M)ₘ(XN)ₙA (IV)

dans laquelle :
- B² répond à une ou plusieurs formules générales (a) ou (b) ci-après : 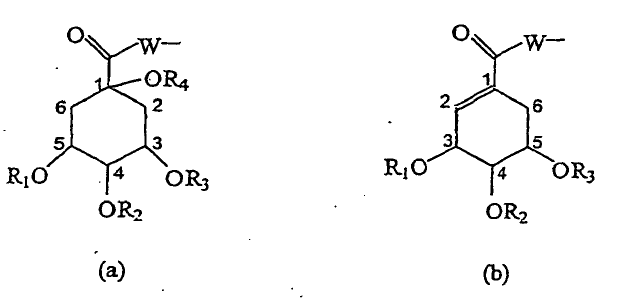 où R₁, R₂, R₃ et R₄ représentent, indépendamment les uns des autres, un atome d'hydrogène ou un groupe protecteur, et où W représente une liaison ou une chaîne carbonée linéaire, ramifiée ou cyclique, saturée ou insaturée, comprenant de 1 à 18 atomes de carbone et éventuellement de 1 à 12 atomes choisis parmi l'oxygène, le soufre et l'azote, ladite chaîne carbonée étant éventuellement substituée par 1 à 16 atomes d'halogène,
- X représente le reste d'un oligopeptide X' comprenant de 1 à 6 acides aminés,
- A représente le reste d'un composé A' au moins trifonctionnel comprenant un enchaînement d'acides aminés, identiques ou différents, sélectionnés dans le groupe constitué par la lysine, l'hydroxylysine, la sérine, la thréonine, la cystéine, l'ornithine, l'acide aspartique et l'acide glutamique
- m est un nombre entier compris entre 1 et 32,
- n est un nombre entier compris entre 0 et 32, et
- M et N représentent chacun un groupe de liaison, respectivement entre B² et A quand n = 0 ou B² et X quand n est différent de 0, et entre X et A quand n est différent de 0, et comprennent, indépendamment l'un de l'autre, une fonction choisie parmi les fonctions oxime, hydrazone, amide, ester, thioester, hydrazide, hydroxamate, éther, thioéther, amine, carbonate, carbamate, thiocarbonate, thiocarbamate, urée, thiourée et thiazolidine.

Le composé de formule (IV) est décrit dans la demande de brevet internationale PCT/FR00/02194.

Au sens de la présente invention, on entend par groupe « protecteur » un groupe protecteur d'une fonction hydroxyle ou d'un diol (auquel cas deux groupes choisis parmi R₁, R₂, R₃ et R₄ peuvent être reliés de façon covalente l'un à l'autre pour former ensemble un cycle), tel que défini dans l'ouvrage *Protective Groups in* Organic Synthesis, T.W. GREENE et P.G.M. WUTS, Seconde Edition 1991, J. WILEY and Sons. A titre d'exemples et de façon non-limitative, on peut citer les groupes triméthylsilyle, triéthylsilyle, triisopropylsilyle, tertiobutyldiméthylsilyle, tertiobutyldiphénylsilyle, triméthylsilyléthyléther, tert-butoxyméthyle, méthoxy-méthyle, benzyloxyméthyle, 2-méthoxyéthoxyméthyle, méthylthiométhyle, acétyle, ou 2',3'-diméthoxybutane-2',3'-diyle.

A titre d'exemples et de façon non-limitative, dans les formules générales (a) et (b) :
- des atomes d'halogène convenables sont le brome, le chlore et le fluor ; et
- des chaînes carbonées convenables sont des groupes alkyles (méthyle, éthyle, propyle, isopropyle, tertiobutyle, pentyle, ...), des groupes alkényles (vinyle, allyle, butényle, pentényle, ...), des groupes alkynyles (éthynyle, propynyle, butynyle, pentynyle, ...), des groupes cycloalkyles (cyclopentyle, cyclohexyle, ...), des hétérocycles (pipérazine, ...), des groupes aryles (phényle, crésyle, ...), des groupes hétéroaryles (pyridine, pyrimidine, pyrazine, ...), des groupes polyéthylèneglycols ou encore des polyamines.

Les formules (a) et (b) représentent les restes des acides quinique et shikimique et de certains de leurs dérivés, obtenus par protection des hydroxyles portés aux positions 1, 3, 4 et/ou 5 du cycle.

Dans un mode de réalisation préféré selon l'invention, le composé de formule générale (IV) est tel que m est un nombre entier compris entre 4 et 16, n est un nombre entier compris entre 2 et 8, X représente le reste d'un oligopeptide comprenant de 2 à 4 résidus d'acides aminés, tandis que A représente le reste d'un enchaînement comprenant de 2 à 8 résidus de lysine et se présentant sous la forme d'un dendrimère, et B² représente un reste d'un ou plusieurs composés choisis parmi l'acide (-)-shikimique et l'acide (-)-quinique.

Dans le mélange de lipopeptides selon la formule (I), la présence de composés peptidiques selon la formule (IV) permet de vectoriser sélectivement les lipopeptides vers les cellules possédant des récepteurs du mannose ou des récepteurs de la famille des C-lectines apparentés au récepteur du mannose. Ainsi, le mélange de lipopeptides comprenant des composés peptidiques P constitués par un antigène peptidique et un glycomimétique dendrimérique selon la formule (IV) permettent de cibler ledit mélange de lipopeptides vers les cellules exprimant les récepteurs de la famille des C-lectines apparentés au récepteur du mannose.

L'utilisation d'un mélange de lipopeptides selon la présente invention comprend avantageusement dé 1 à 20 lipopeptides différents, de préférence de 1 à 10 lipopeptides différents.

L'utilisation selon la présente invention de mélange de lipopeptides pour la fabrication de vaccins s'entend dans le sens de l'utilisation pour la fabrication de tous les types de vaccins, c'est à dire de manière classique les vaccins prophylactiques, mais également les « vaccins à usage thérapeutique ». Ces vaccins peuvent être dirigés contre les maladies infectieuses d'origine bactérienne, virale ou parasitaire ou contre différents types de cancers. L'utilisation selon l'invention permet aussi la fabrication de vaccins polyvalents comprenant le mélange d'antigènes peptidiques correspondant à différentes maladies infectieuses.

Les vaccins selon la présente invention sont donc non seulement actifs dans une optique de vaccination préventive mais également dans une optique thérapeutique en particulier les vaccins lipopeptidiques selon la présente invention peuvent être utilisés pour créer une réponse contre de nouveaux épitopes ainsi que pour polariser ou renforcer la polarisation d'une réponse immunitaire établie. Les résultats observés indiquent en effet que des lipopeptides sont capables d'induire de nouvelles réponses immunitaires anti VIH, CD4, CD8 chez les patients infectés.

Le composé peptidique est avantageusement constitué par des antigènes peptidiques dérivés d'un agent infectieux de type viral, tel que le VIH ou le VHC.

L'utilisation pour la fabrication d'un vaccin d'un mélange comprenant au moins des lipopeptides selon l'invention est formulée de manière préférentielle pour l'administration par voie transmucosale ou transcutanée, mais également parentérale (intra-musculaire, sous-cutanée, intradermique, intra-veineuse).

La plupart des vaccins actuellement disponibles sont administrés par voie parentérale, ce qui implique un personnel médical formé, des coûts élevés, peut provoquer des réactions au site d'injection, et dans certaines circonstances est à l'origine d'infections suite à l'utilisation de seringues contaminées (ex : transmission VIH, virus des hépatites). L'OMS a ainsi estimé que, dans les pays en voie de développement, 50% des injections effectuées dans une optique de vaccination ne sont pas sécurisées en raison d'une large réutilisation de seryngues non stériles (Jodar L. et al., Vaccine, 19 (2001), 1594-1605 ; Kane A. et al., Bull WHO 77 (1999) 801-807). De plus, l'utilisation d'aiguilles est en général, pour les enfants, synonyme de souffrance. Il apparaît donc nécessaire de développer une nouvelle génération de vaccins, faciles à administrés, économiques, avec des protocoles d'immunisation aisés à mettre en oeuvre.

Les vaccins mucosaux et transcutanés représentent une alternative attractive aux vaccins administrés par voie parentérale. L'administration mucosale, par exemple par la voie nasale, permet d'induire des réponses immunes au niveau de sites qui sont la porte d'entrée de nombreux pathogènes, et constitue une alternative efficace à l'administration par voie orale laquelle reste associée à un faible accès aux cellules présentatrices d'antigènes, la dégradation protéolytique siégeant au niveau du tractus intestinal et donc une réponse immunitaire de faible amplitude.

La peau, principale interface avec l'environnement externe, est aussi une barrière immunitaire contre l'entrée de nombreux pathogènes. Elle présente des cellules immunocompétentes spécifiques que sont les kératinocytes et surtout les cellules de Langerhans, est équipée de ganglions lymphoïdes, ainsi que de sous-classes de lymphocytes T, l'ensemble constituant « le tissue lymphoïde associé à la peau » (Skin-associated lymphoid tissue, SALT) (Bos J.D., et al., Immunol. Today, 14, (1993), 75-78).
Leur capacité à franchir les membranes cellulaires permet aux lipopeptides de véhiculer des épitopes à travers les surfaces muqueuses, permettant ainsi leur délivrance non seulement au niveau du système immunitaire local, mais également au niveau systémique (Demande Internationale N°WO 0141797).

L'utilisation pour la fabrication d'un vaccin d'un mélange comprenant au moins des lipopeptides, selon la présente invention, comprend en outre des constituants à caractère amphiphile ou excipients capables d'assurer une bonne solvatation dans l'eau par une partie polaire et une interaction de type Van der Waals avec les lipopeptides à caractère hydrophobe:

Ces excipients ou constituants amphiphiles peuvent être constitués, pour une majeure partie, d'acides aminés polaires ou chargés.

Dans un mode de réalisation préféré selon l'invention, le mélange de lipopeptides comprend en outre un ou plusieurs excipients cliniquement acceptables.

D'une manière particulièrement avantageuse, l'excipient cliniquement acceptable est le mannitol ou le Polysorbate 80, ou un mélange des deux.

La présente invention concerne notamment les vaccins, comportant en tant qu'antigènes peptidiques, au moins deux séquences provenant des protéines Gag, Pol et Nef du virus HIV, notamment un mélange des séquences Gag 17-35, Gag 253-284, Pol 325-355, Nef 66-97, Nef 116-147 et plus particulièrement les lipopeptides tels que décrits dans l'exemple 1.

Dans les lipopeptides utilisés, on introduit de préférence une lysine supplémentaire à l'extrémité C-terminale de chaque peptide, et l'acide carboxylique en C-terminale est amidé. La fonction amine (e-NH2) de la chaîne latérale de cette lysine est modifiée par une moitié palmitoyl attachée via un espaceur contenant une liaison hydrazone.

Dans encore un autre mode de réalisation préféré, l'utilisation selon l'invention comprend en tant qu'antigènes peptidiques, les séquences peptidiques suivantes :
- VHC 1 HzAc : H-K(COCH₂NHNH₂)GREILLGPADGMVSKGWRLLAPITAYAQQTR-NH2
- VHC 2 HzAC : H-K(COCH₂NHNH₂)GHAVGIFRAAVCTRGVAKAVDF-NH2
- VHC 3 HzAc : H-K(COCH₂NHNH₂)GAAWYELTPAETTVRLRAYMNTPGLPVAQD-NH2.

L'invention concerne également les séquences natives :
NS3 1007-1037 : H-KGREILLGPADGMVSKGWRLLAPITAYAQQTR-NH2
NS3 1174-1195 : H-KGHAVGIFRAAVCTRGVAKAVDF-NH2
NS3 1524-1553 : H-KGAAWYELTPAETTVRLRAYMNTPGLPVAQD-NH2,
   lesquelles présentent donc un caractère immunogène.

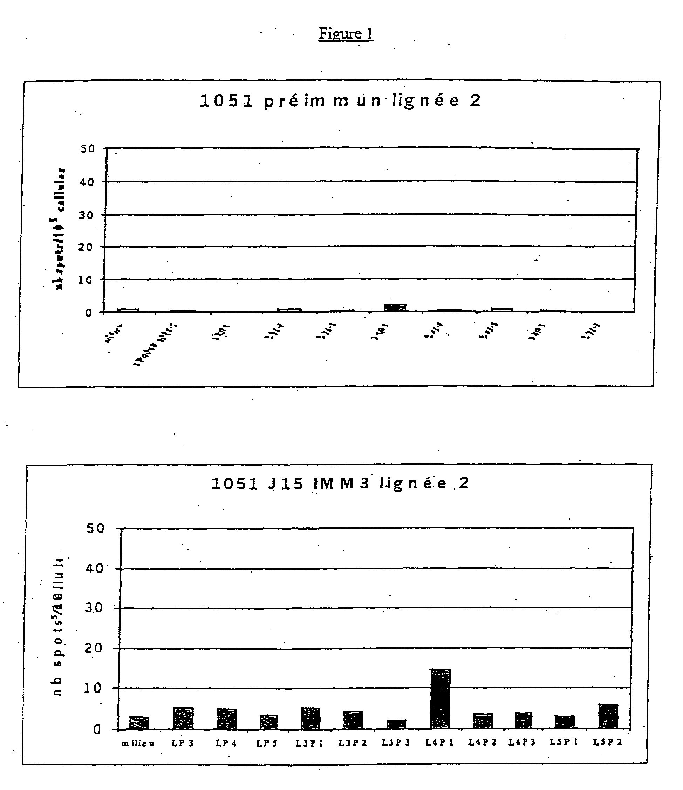

Outre les dispositions qui précèdent, l'invention comprend encore d'autres dispositions qui ressortiront de la description qui va suivre, qui se réfère à des exemples de mise en oeuvre de l'utilisation pour la fabrication d'un vaccin d'un mélange comprenant au moins des lipopeptides selon l'invention, ainsi qu'à la figure 1 qui illustre les résultats d'analyse de l'immunogénicité chez le macaque après l'injection de doses de vaccin (VIS) selon l'exemple 5.

Dans les exemples qui suivent, les abréviations suivantes sont utilisées : eq. : équivalents ; Boc : tert-butyloxycarbonyle ; Fmoc : 9-fluorènyl-méthoxycarbonyle ; Mtt : 4-méthyl-trityle ; DMF : diméthylformamide ; TFA : acide trifluoroacétique ; CH₂Cl₂ : dichlorométhane ; MeOH : méthanol ; EtOH : éthanol ; THF : tétrahydrofurane ; AcOH : acide acétique ; AcOEt : acétate d'éthyle ; H₂O : eau ; AcO⁻NH₄⁺ : acétate d'ammonium ; CDCl₃ : chloroforme deutéré ; EDT : éthanedithiol ; TIS : triisopropylsilane ; DMSO-*d6* : diméthylsulfoxyde-d6 (entièrement deutéré) ; NaIO₄ : periodate de sodium ; NaCl : chlorure de sodium ; Na₂SO₄ : sulfate de sodium ; MgSO₄ : sulfate de mag-nésium ; KH₂PO₄ : dihydrogénophosphate de potassium ; PAL : « peptide-amide linker »; Bop : benzotriazole-1-yl-oxy-tris(diméthylamino)phosphoniumhexafluorophosphate ; HOBt : N-hydroxybenzotriazole ; HBTU : N-oxyde d'hexafluorophosphate de N-[(1H-benzotriazol-1-yl)(diméthylamino)méthylène]-N-méthylméthanaminium ; DIEA : diisopropyléthylamine ; TEAP : phosphate de triéthylamine ; HPLC : chromatographie liquide haute performance ; RP-HPLC : chromatographie liquide haute performance en phase inverse ; ES-MS : spectrométrie de masse par électronébulisation ; TOF-PDMS : spectrométrie de masse à désorption par plasma ; LC-MS : spectrométrie de masse couplée à une analyse par chromatographie liquide ; RMN ¹H : résonance magnétique nucléaire du proton ; RMN ¹³C : résonance magnétique nucléaire du carbone.

### Exemple 1 Préparation de doses de vaccin (lipopeptides HIV-HzPAM) utilisables chez l'Homme, Modèle du Virus de l'Immunodéficience Humaine (VIH-1)

### 1) Synthèse des hydrazinopeptides

### Synthèse peptidique :

Cinq peptides, dérivés des protéines Gag, Pol et Nef du virus HIV-1, fonctionnalisés par des groupes α-hydrazinoacétiques au niveau de la chaîne latéral d'une lysine introduite en C-terminal, ont été préparés. Ces hydrazinopeptides ont été synthétisés en phase solide sous forme de sels de trifluoroacétate. Les stratégies Fmoc/*tert*-Butyl (conventionnelle), ainsi que le procédé qui a été récemment décrit (N,N',N'-tri(tert-butyloxycarbonyl)-hydrazinoacetic acid) ont été employés pour introduire de manière ciblée un groupement hydrazinoacétyl sur un groupement ε-amino déprotégé d'un résidus lysylamide en position C-terminale (BONNET D. et coll., J. Org. Chem, 2001 et demande de brevet PCT/FR00/02336). Selon les séquences, les rendements de synthèse sont compris entre 10 et 50 %. L'identité des peptides a été vérifiée, après clivage et déprotection, par ES.MS. Les hydrazinopeptides sont soumis à une analyse de la composition en acides aminés après hydrolyse acide totale avec de l'acide chlorhydrique 6N HC1:phenol, 10:1 à 110°C pendant 24h.

### Chroma tographie

Les hydrazinopeptides sont purifiés par RP-HPLC sur une colonne préparative C3 (colonne Zorbax : 300 Å, 5 µM, diamètre de 12,5 mm et longueur de 250 mm avec précolonne) sur une chaîne HPLC Shimatzu 6A. Les analyses par RP-HPLC sont réalisées sur une colonne préparative C3 (colonne Zorbax : 300 Å, 5 µM, diamètre de 4.6 et longueur de 250 mm) sur une chaîne HPLC Shimatzu 10A.

Les solvants d'élution (solvants A et B) sont les suivants :
- solvant A : eau désionisée comprenant 0,05% de TFA,
- solvant B (purification) : 0,05% de TFA dans un mélange propan-2-ol / H₂O (40:60).
- solvant B (suivi de réaction, Analyses) : 0,05% de TFA dans un mélange acétonitrile / H₂O (80:20).

Le débit d'élution est de 1ml/min en utilisant un gradient linéaire de 10 à 100 % de tampon B en 30 minutes, 100 % de tampon B pendant 10 minutes, 10 % de tampon B pendant 10 minutes (détection à 215 nm, température : 50°C).

Les peptides obtenus, sous forme de sels de trifluoroacétate, sont représentés dans le Tableau 1 ci-après.

**Tableau 1 : Hydrazinopeptides dérivés de protéines du virus VIH-1**

| Peptides natifs | Hydrazinopeptides |
|---|---|
| Gag 17 - 35 | VIH 1 HzAc : EKIRLRPGGKKKYKLKHIV K(COCH₂NHNH₂)-NH₂ |
| Gag 253 - 284 | VIH 2 HzAc : NPPIPVGEIYKRWIILGLNKIVRMYSPTSILD K(COCH₂NHNH₂)-NH₂ |
| Pol 325 - 355 | VIH 3 HzAc : AIFQSSMTKILEPFRKQNPDIVIYQYMDDLY K(COCH₂NHNH₂) -NR₂ |
| Nef 66 - 97 | VIH 4 HzAc : VGFPVTPQVPLRPMTYKAAVDLSHFLKEKGGL K(COCH₂NHNH₂)-NH₂ |
| Nef 116 - 147 | VIH 5 HzAc : HTQGYFPDWQNYTPGPGVRYPLTFGWLYKLVP K (COCH₂NHNH₂) -NH₂ |

Les temps d'élution des hydrazinopeptides obtenus sont représentés dans le Tableau II suivant.

**Tableau II**

| Hydrazino peptides | Temps de rétention (minutes) | [M+H]⁺ calculé | [M+H]⁺ trouvé |
|---|---|---|---|
| VIH 1 HzAc | 6.46 | 2490.6 | 2490.4 |
| VIH 2 HzAc | 17.15. | 3896.2 | 3895.1 |
| VIH 3 HzAc | 15.11 | 3966.0 | 3966.0 |
| VIH 4 HzAc | 13.86 | 3695.0 | 3695.0 |
| VIH 5 HzAc | 14.86 | 3800.8 | 3800.8 |

### 2) Synthèse du vecteur lipophile Pam (acide [3-(2-oxo-acetylamino)-propyl]-amide Hexadecanoique, présent également sous forme "Vecteur Pam hydraté" : acide [3-(2,2-dihydroxy-acetylamino)-propyl]-amide Hexadecanoiue :

La synthèse de ce vecteur, composé d'un groupement palmitoyl et d'un groupement glyoxylyl, a été décrite par MELNYK et collaborateurs dans la demande de brevet N° PCT/FR01/02787 (exemple 4 : synthèse du vecteur lipophile « IIIa ») et par Bonnet et collaborateurs (BONNET D et coll., Tetrahedron Letters, 2000).

Dans un ballon de 500 ml, 10 g (66,6 mmoles) d'acide tartrique 1 sont dissous dans 200 ml d'éthanol absolu, en présence de 1% en masse de résine Amberlyst 15. Le ballon est muni d'un montage soxhlet contenant du tamis moléculaire activé 4 A. L'éthanol est porté à reflux durant 48h. Le milieu réactionnel est ensuite filtré sur verre fritté n°4 pour éliminer la résine. Le filtrat est concentré sous pression réduite et conduit à une huile jaune-clair. Le résidu 2 est séché une nuit sur P₂O₅ et utilisé sans autre forme de purification (m = 13,46 g, rendement = 98,0 %).

L'analyse du composé 2, représenté ci-dessous, par chromatographie sur couche mince donne un indice de migration Rf = 0,76 dans l'éluant CH₂Cl₂/MeOH/H₂O/AcOH (90/10/0, 1/0, 05).

RMN ¹H (CDCl₃) : 1, 33 (t, 6H, H₅,_{5'}, J₅₋₄=7,2 Hz), 3,28 (s, 2H, H_{1,1'}), 4,33 (q, 4H, H₄,_{4'}, J₄₋₅=7,1 Hz), 4,54 (s, 2H, H₂,_{2'})- RMN ¹³C : 14, 14 (C₅,_{5'}), 62,48 (C_{4,4'}). 72, 08 (C₂,_{2'}), 171,62 (C_{3,3'}).

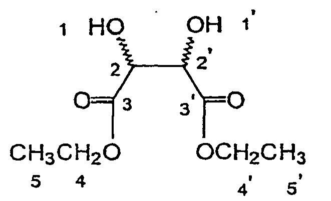

13,46 mg (65,26 mmoles) du composé 2 sont dissous dans 155 ml d'éthanol absolu et ajoutés goutte à goutte à 56 ml (665,26 mmoles) de 1,3-diamino-propane. Le milieu réactionnel est ensuite agité 3h à température ambiante. L'excès de 1,3-diaminopropane est alors éliminé par évaporation sous pression réduite et entraînement azéotropique en présence d'éthanol (3 x 200 ml). L'huile jaune obtenue est séchée sur P₂O₅ pendant une nuit, puis reprise dans un mélange éthanol/toluène (1/1) et concentrée sous pression réduite (3 x 200 ml). Un composé jaune, sous une forme pâteuse, est ainsi obtenu. Ce composé est précipité dans 50 ml d'un mélange éther éthylique/éthanol (5/1), puis filtré, et le précipité blanchâtre est agité pendant 1h30 à 0°C en présence d'éther éthylique. Il est alors filtré sur fritté et lavé 2 fois avec le minimum d'éther éthylique à froid. Le solide résiduel 3 est séché pendant une nuit sur P₂O₅ (m = 13,92 g, rendement = 81,3%).

L'analyse du composé 3, représenté ci-dessous, est la suivante : Rf = 0,34 dans THF/AcOH/H₂O/AcO⁻NH₄⁺ (35/20/10/1% massique) RMN ¹H (DMSO-d6): 1,48 (q, 4H, H_{6 et 6'}, J_{6-5,6,7 et 6'-5',6',7'}=6,65 H₂), 2,51 (m, 4H, H_{7-7'}), 3,14 et 3,16 (t, 4H, H_{5 et 5'}, J_{5-6 et 5'}-_{6'}=6,3 Hz), 4,19 (s, 2H, H_{2,2}), 7,75 (t, 2H, H₄,_{4'}, J₄,_{4'}= 5,97 Hz). RMN ¹³C : 33, 51 (C₆,_{6'}), 36,98 (C_{7,7'}), 40,06 (C₅,_{5'}), 73,43 (C₂,_{2'}), 172, 87 (C_{5,5'}).

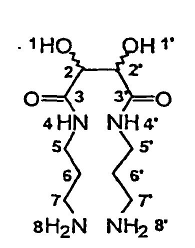

996,9 mg (3,8 mmoles) du composé 3 sont dissous dans 15 ml d'eau. Le pH de la solution, égal à 8,5, est ramené à 3,25 à l'aide de 1,46 g d'acide citrique. 1,06 g (4,9 mmoles) de NaIO₄ solide sont alors ajoutés en quatre fois, pendant 5 minutes, au milieu réactionnel maintenu à température ambiante à l'aide d'un bain d'eau. Après 10 minutes d'agitation, 570 mg d'acide tartrique (3,8 mmoles) sont ajoutés pour neutraliser l'excès de NaIO₄ n'ayant pas réagi. Après 10 minutes d'agitation à température ambiante, le pH est ajusté à 8,6 avec 24 ml de N-méthyl-morpholine. Le milieu réactionnel est dilué avec 70 ml de 2-méthyl-propan -2-ol et 20 ml d'eau. 2,16 g (1,6 eq.) de palmitoate de succinimidyle sont alors ajoutés à la solution qui est agitée une nuit à température ambiante. Le pH est ensuite ajusté à 3,5 avec 7,11 g d'acide citrique. Le milieu est dilué avec 40 ml d'une solution saturée en NaCl et extrait avec 140 ml de dichlorométhane, puis 100 ml de chloroforme. La phase organique est lavée avec deux fois 100 ml d'une solution saturée en KH₂PO₄, deux fois 100 ml d'une solution saturée en NaCl, puis séchée sur Na₂SO₄, filtrée et concentrée sous pression réduite. Le composé solide résiduel est purifié sur silice en utilisant comme éluant un mélange CH₂Cl₂/AcOEt/EtOH (70/22/5). 951,4 mg du vecteur lipophile (IIIa), soit un rendement de 42,3%, sont ainsi obtenus.

L'analyse du composé (IIIa), représenté ci-dessous sous sa forme d'hydrate, est la suivante : RMN ¹H (DMSO-d6): 0,87 (t, 3H, H₁, J_{1,2}=5,31 Hz), 1,25 (m, 24H, H₂) , 1,83 (m, 4H, H₃, H₈), 2,39 (m, 2H, H₄), 3,57 (m, 4H, H₇, H₉), 4,41 (s, 2H, H_{14,15}), 5,50 (m, 1H, H₁₂). RMN ¹³C : 14,31 (C₁) , 22,96 (C₂), 26, 35 (C₈), 29,99 (C₃), 36, 79 (C₄), 37,14 (C₇), 92,18 (C₁₂), 173,42 (C₅), 175, 22 (C₁₁). ES-MS : [M+H]⁺ calculé 387,5 ; trouvé 387, 2.

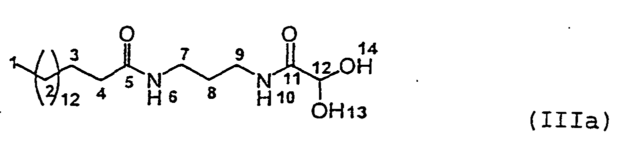

### 3) Synthèse des lipopeptides

Un mélange homogène d'hydrazinopeptides est obtenu, par dilution individuelle de chaque ingrédient dans l'eau ou l'acide acétique dilué, mélange des solutions puis lyophilisation. Une première synthèse a été réalisée en utilisant 9, 6 mg d' un mélange équimassique de chacun des 5 peptides VIH (VIH 1 HzAc; VIH 2 HzAc; VIH 3 HzAc; VIH 4 HzAc et VIH 5 HzAc), correspondant à une valeur de 2,22 micromoles de peptides (en tenant compte des contre-ions et de la teneur en peptides des lyophilisats). Ce mélange est imprégné de 85 µl d'eau, ce qui conduit à la formation d'un gel. Le vecteur lipophile IIIa est alors ajouté (977 µg, 2,52 µmoles) sous agitation magnétique avec des billes de verre de 5 mm de diamètre, soit 1,14 équivalent par rapport aux peptides, en solution dans 714 ml de 2-méthyl-propan-2-ol- Le volume total de milieu réactionnel (1,7 ml) est atteint par addition de 2-méthyl-propan-2-ol.

La disparition des hydrazinopeptides au cours du temps est suivie parallèlement à l'apparition des lipopeptides correspondants.

Une expérience micropréparative est réalisée afin de permettre l'identification des produits : un échantillon est prélevé pour réaliser une séparation des constituants par RP-HPLC sur une colonne C3 (Zorbax, 4,6 mm x 250 mm), avec collecte des pics principaux afin de les identifier par spectrométrie de masse par MALDI-TOF (MALDI-TOF Voyager ABI 4192, mode positif, réflecteur 25 kV, délai d'extraction 300 ms, matrice acide α-cyano cinnmique)

Après 4 heures la réaction est complète et le mélange est alors dilué dans l'eau, congelé et lyophilisé. Les lipopeptides obtenus se présentent sous forme de poudre blanche.

Les temps de rétention (rét.) des lipopeptides obtenus sont donnés dans le Tableau III suivant.

**TABLEAU III**

| Lipopeptide | Temps de | [M+H]⁺ | [M+H]⁺ |
|---|---|---|---|
| VIH 1 HzPam | 15.99 | 2840.5 | 2840.7 |
| VIH 2 HzPam | 21.6 | 4246.2 | 4244.0 |
| VIH 3 HzPam | 19.96 | 4316.0 | 4315.6 |
| VIH 4 HzPAM | 18.48 | 4045.0 | 4044.9 |
| VIH 5 HzPAM | 20.82 | 4150.9 | 4150.2 |

### 4°) Préparation des mélanges de lipopeptides

### Echange d'ions :

Les lipopeptides sous forme de sels de TFA sont dissous dans une solution d'acide acétique aqueux à pH3, chargés sur colonne de RP-HPLC (colonne Nucleoprep C4, 25 mm x 100 mm) puis lavés par le même solvant (lavages effectués avec un volume équivalent à celui de 5 colonnes) avant d'être élués par un passage sans transition vers un solvant acide acétique à pH3 dans l'eau / propan-2-ol (60:40).

### Préparation finale:

Le mélange de lipopeptides lyophilisés est dissous dans une solution d'acide acétique à 10 % avant d'être soumis à une filtration stérilisante sur membrane 0,22 micron, (SLGV, Millipore).

### 5) Préparation des doses de vaccin:

Après l'introduction d'excipients cliniquement acceptables (mannitol / lipopeptides 10/1 (w/w) et Polysorbate 80 / lipopeptides 2/7 (w/w)) la solution est diluée dans l'eau et aliquotée dans des ampoules de verre stériles avant lyophilisation finale.

Les doses unitaires de vaccin préconisées pour un essai clinique de phase I chez l'homme sont de 2,5 mg de coktail lipopeptidique.

### Exemple 2 : Préparation de doses de vaccin (lipopeptides VHC HzPam) utilisables chez l'homme, Modèle du Virus de l'Hépatite C (VHC)

### 1) Synthèse des hydrazinopeptides

### Synthèse peptidique :

Trois peptides, dérivés de la protéine NS3 du virus VHC-1, fonctionnalisés par des groupes α-hydrazinoacétiques au niveau de leur extrémité N-terminale ont été préparés. Ces hydrazinopeptides ont été synthétisés en phase solide sous forme de sels de trifluoroacétate. Les stratégies Fmoc/tert-Butyl (conventionnelle), ainsi que le procédé récemment décrit (N,N',N'-tri(tert-butyloxycarbonyl)-hydrazinoacetic acid) ont été employés pour introduire de manière ciblée un groupement hydrazinoacétyl sur un groupement ε-amino déprotégé d'un résidu lysylamide en position N-terminale comme dans l'exemple 1. Selon les séquences, les rendements de synthèse sont compris entre 10 et 50 %. L'identité des peptides a été vérifiée par ES.MS. Les hydrazinopeptides sont soumis à une analyse de là composition en acides aminés après hydrolyse acide totale avec de l'acide chlorhydrique 6N HCl:phenol, 10:1 à 110°C pendant 24h.

### Chromatographie

Les hydrazinopeptides sont purifiés par RP-HPLC sur une colonne préparative C3 (colonne Zorbax : 300 Å, 5 µM, diamètre de 12, 5 mm et longueur de 250 mm avec précolonne) sur une chaîne HPLC Shimatzu 6A. Les analyses par RP-HPLC sont réalisées sur une colonne préparative C3 (colonne Zorbax :300 Å, 5 µM, diamètre de 4.6 et longueur de 250 mm) sur une chaîne HPLC Shimatzu 10A.

Les solvants d'élution (solvants A et B) sont les suivants :
- solvant A : eau désionisée comprenant 0,05% de TFA,
- solvant B (purification) : 0,05% de TFA dans un mélange propan-2-ol / H₂O (40:60).
- solvant B (suivi de réaction) : 0,05% de TFA dans un mélange N propanol / H₂O (40:60).

Le débit d'élution est de 1ml/min en utilisant un gradient linéaire de 0 à 100 % de tampon B en 30 minutes, 100 % de tampon B pendant 5 minutes, 0 % de tampon B pendant 10 minutes (détection à 215 nm, température : 50°C).

Les hydrazinopeptides obtenus sont représentés dans le tableau IV ci-après.

**TABLEAU IV : Hydrazinopeptides dérivés de la protéine NS3 du virus VHC-1**

| **Peptides natifs** | **Hydrazinopeptides** |
|---|---|
| NS3 1007-1037 | VHC 1 HzAc : H-K(COCH₂NHNH₂)GREILLGPADGMVSKGWRLLAPITAYAQQTR-NH2 |
| NS3 1174-1195 | VHC 2 HzAc : H-K(COCH₂NHNH₂)GHAVGIFRAAVCTRGVAKAVDF-NH2 |
| NS3 1524-1553 | VHC 3 HzAc : H-K (COCH₂NHNH₂) GAAWYELTPAETTVKLRAYMNTPGLPVAQD-NH2 |

Les temps de rétention des hydrazinopeptides sont indiqués dans le Tableau V ci-après.

**Tableau V**

| **Hydrazinopeptides** | **Temps de rétention (minutes)** | **[M+H] ⁺ calculé** | **[M+H] ⁺ trouvé** |
|---|---|---|---|
| VHC 1 HzAc | 17.86 | 3567.9 | 3567.9 |
| VHC 2 HzAc | 13.9 | 2444.3 | 2444.3 |
| VHC 3 HzAc | 16.66 | 3490.7 | 3490.8 |

### 2) Synthèse du vecteur Pam :

La synthèse de ce vecteur IIIa, composé d'un groupement palmitoyl et d'un groupement glyoxylyl, est décrite dans l'exemple 1.

### 3) Synthèse des lipopeptides

Un mélange homogène d'hydrazinopeptides est obtenu par dilution individuelle de chaque ingrédient dans l'eau ou l'acide acétique dilué, mélange des solutions puis lyophilisation. Une première synthèse a été réalisée en utilisant 41,8 mg d'un mélange équimassique de chacun des 3 hydrazinopeptides, correspondant à une valeur de 10,77 micromoles d'hydrazinopeptides (en tenant compte des contre-ions et de la teneur en peptides des lyophilisats). Ce mélange est imprégné de 415 µl d'eau, ce qui conduit à la formation d'un gel. Le vecteur lipophile IIIa est alors ajouté (4757 µg, 12,28 µmoles, représentant 1,14 équivalent par rapport aux peptides) sous agitation magnétique avec quelques billes de verre de 5 mm de diamètre, solubilisé dans 950 µl de 2-méthyl-propan-2-ol. Le volume total de milieu réactionnel (8.290 ml) est atteint par addition de 2-méthyl-propan-2-ol.

La disparition des hydrazinopeptides au cours du temps est suivie parallèlement à l'apparition des lipopeptides correspondants.

Une expérience micropréparative est réalisée afin de permettre l'identification des produits : un échantillon est prélevé pour réaliser une séparation des constituants par RP-HPLC sur une colonne C3 (Zorbax, 4,6 mm x 250 mm), avec collecte des pics principaux afin de les identifier par spectrométrie de masse par MALDI-TOF (MALDI-TOF Voyager ABI 4192, mode positif, réflecteur, 25 kV, délai d'extraction 300 ms, matrice acide α-cyano cinnamique)

Après 4 heures la réaction est complète et le mélange est alors dilué dans l'eau, congelé et lyophilisé. Les lipopeptides obtenus se présentent sous forme de poudre blanche.

Les lipopeptides obtenus ont un temps de rétention présentés dans le Tableau VI ci-après.

**TABLEAU VI**

| Lipopeptides VHC HzPam | Temps de rétention (minutes) | [M+H] ⁺ calculé | [M+H]⁺ trouvé |
|---|---|---|---|
| VHC 1 HzPam | 23.9 | 3917.9 | 3917.07 |
| VHC 2 HzPam | 25.1 | 27.94.3 | 2793.9 |
| VHC 3 HzPam | 25.1 | 3840.7 | 3840.9 |

### 3') Synthèse des lipopeptides avec un glycomimétique branché :

Cette synthèse est réalisée à partir du mélange des hydrazinopeptides VHC obtenus précédemment avec un glycomimétique branché à quatre valences *(arbre tetraquinoylé)* réalisé à partir d'un arbre de lysine synthétisé en stratégie Fmoc/tert Butyl sur lequel a été introduit un groupement hydrazinoacetyl sur le groupement ε-amino déprotégé du résidu lysylamide C-terminale, selon la formule (IV) de l'invention.

La synthèse de ce glycomimétique, appelé [(Qui) ₄AKHdz)], est décrite dans l'exemple 2 de la demande de brevet N° PCT/FR01/02787).

Un mélange homogène d'hydrazinopeptides est obtenu par dilution individuelle de chaque ingrédient dans l'eau ou l'acide acétique dilué, mélange des solutions puis lyophilisation. 8,9 mg d'un mélange équimassique de chacun des 3 hydrazinopeptides, correspondant à 2,14 micromoles d'hydrazinopeptides sont ajoutés à 2,3 mg (soit 1.6 µmoles) du glycomimétique hydrazine. Au gel formé après humidification par 140 µl d'eau est ajouté le vecteur lipophile IIIa (1640 microgrammes, soit 1,14 équivalent peptide) mis en solution dans le 2-méthyl-propan-2-ol (410 µl), sous agitation magnétique avec quelques billes de verre de 5 mm de diamètre, le volume final est de 2,7 ml.

La disparition des hydrazinopeptides au cours du temps est suivie parallèlement à l'apparition des lipopeptides correspondants.

Après 3 heures, le mélange est dilué dans l'eau, congelé et lyophilisé. Les lipopeptides obtenus se présentent sous forme de poudre blanche.

### 4) et 4') Préparation des mélanges de lipopeptides

Après échange d'ions comme décrit dans l'exemple 1, les mélanges de lipopeptides lyophilisés obtenus en 3) et 3') sont dissous dans une solution d'acide acétique à 10 % avant d'être soumis à une filtration stérilisante sur membrane 0,22 micron (SLGV, Millipore).

### 5) et 5') Préparation des doses de vaccin

### 5) Formulation mannitol - Tween^{®} : [3 lipopeptides, vecteur Pam]

Après l'introduction d'excipients cliniquement acceptables (mannitol / lipopeptides 10/1 (w/w) et Polysorbate 80 / lipopeptides 2/7 (w/w)) la solution est diluée dans l'eau et aliquotée dans des ampoules de verre stériles avant lyophilisation finale.

### 5') Formulation mannitol - glycomimétique lipidé : [3 lipopeptides + glycomimétique, vecteur Pam]

Le mélange lyophilisé de lipopeptides et glycomimétique lipidé [(Qui)₄AKHdz)] est dissous dans une solution d'acide acétique à 10 % avant d'être soumis à une filtration stérilisante sur membrane 0,22 micron (SLGV, Millipore). Après l'introduction d'excipients cliniquement acceptables (mannitol / lipopeptides 10/1 (w/w)), la solution est diluée dans l'eau dans un flacon stérile avant lyophilisation finale.

Les doses unitaires de vaccin préconisées pour un essai clinique de phase I chez l'homme sont de 2,5 mg de cocktail lipopeptidique.

### Exemple 3 : Préparation de doses de vaccin (lipopeptides VHC HzCChol) utilisables chez l'homme, Modèle du Virus de l'Hépatite C (VHC)

### 1) Synthèse des hydrazinopeptides

### Synthèse peptidique

La synthèse est la même que celle de l'exemple 2.

### Chromatographie

Les hydrazinopeptides sont purifiés par RP-HPLC sur une colonne préparative C3 (colonne Zorbax : 300 Å, 5 µM, diamètre 12,5 mm et longueur de 250 mm avec précolonne) sur une chaîne HPLC Shimatzu 6A. Les analyses par RP-HPLC sont réalisées sur une colonne préparative C3 (colonne Zorbax : 300 Å, 5 µM, diamètre de 4.6 et longueur de 250 mm) sur une chaîne HPLC Shimatzu 10A. Les solvants d'élution (solvants A et B) sont les suivants :
- solvant A : eau désionisée comprenant 0,05% de TFA,
- solvant B : 0,05% de TFA dans un mélange propan-2-ol / H₂O (40:60).

Le débit d'élution est de 1ml/min en utilisant un gradient linéaire de 0 à 100 % de tampon B en 30 minutes, 100 % de tampon B pendant 5 minutes, 0 % de tampon B pendant 10 minutes (détection à 215 nm, température : 50°C).

### 2) Synthèse du vecteur CChol (acide [3-(2,2-Dihydroxy-acetylamino)-propyl]-carbamique 17-(1,5-dimethyl-hexyl)-10,13-dimethyl-2, 3, 4, 7, 8, 9, 10, 11, 12, 13, 14, 15, 16, 17-tetradecahydro-1H-cyclopenta [a] phenanthren-3-yl ester, également sous forme "Vecteur Cchol hydraté" acide [3-(2-Oxo-acetylamino)-propyl]-carbamique 17-(1,5-dimethyl-hexyl)-10,13-dimethyl-2,3,4,7,8, 9, 10, 11, 12, 13, 14, 15, 16, 17-tetradecahydro-1H-cyclopenta[a] phenan-thren-3-yl ester)

La synthèse de ce vecteur a été décrite par MELNYK et collaborateurs dans la demande de brevet N° PCT/FR01/02787, exemple 4 : synthèse du vecteur lipophile « IIIc », correspondant dans la présente invention à la formule (II).

### 3) Synthèse des lipopeptides

Un mélange homogène d'hydrazinopeptides est obtenu par dilution individuelle de chaque ingrédient dans l'eau ou l'acide acétique dilué, mélange des solutions puis lyophilisation. Une première synthèse a été réalisée en utilisant 12,2 mg d'un mélange équimassique de chacun des 3 peptides VHC (VHC 1 HzAc ; VHC 2 HzAc et VHC 3 HzAc ), correspondant à 2,96 micromoles de peptides (en tenant compte des contre-ions et de la teneur en peptides des lyophilisats). Ce mélange est imprégné de 115 µl d'eau, ce qui conduit à la formation d'un gel. Le vecteur lipophile II est alors ajouté (1926 µg, 3.374 µmoles, représentant 1,14 équivalent par rapport aux peptides) sous agitation magnétique avec quelques billes de verre de 5 mm de diamètre, solubilisé dans 292 µl de 2-méthyl-propan-2-ol. Le volume total de milieu réactionnel (2.3 ml) est atteint par addition de 2-méthyl-propan-2-ol.

La disparition des hydrazinopeptides au cours du temps est suivie parallèlement à l'apparition des lipopeptides correspondants.

Une expérience micropréparative est réalisée afin de permettre l'identification des produits : un échantillon est prélevé pour réaliser une séparation des constituants par RP-HPLC sur une colonne C3 (Zorbax, 4,6 mm x 250 mm), avec collecte des pics principaux afin de les identifier par spectrométrie de masse par MALDI-TOF (MALDI-TOF Voyager ABI 4192, mode positif, réflecteur, 25 kV, délai d'extraction 300 ms, matrice acide a-cyano cinnamique)

Après 4 heures la réaction est complète et le mélange est alors dilué dans l'eau, congelé et lyophilisé. Les lipopeptides obtenus se présentent sous forme de poudre blanche.

Le temps de rétention des lipopeptides obtenus sont représentés dans le Tableau VII ci-après.

**TABLEAU VII**

| **Lipopeptides VHC HzCChol** | **Temps de rétention (minutes)** | **[M+H]⁺ calculé** | **[M+H]⁺ trouvé** |
|---|---|---|---|
| VHC 1 HzCChol | entre 32.2 et 34.2min | 4091.9 | 4091.9 |
| VHC 2 HzCChol | | 2968.3 | 2968.4 |
| VHC 3 HzCChol | | 4014.7 | 4014.8 |

### 3') Synthèse des lipopeptides CChol avec un glycomimétique branché :

Un mélange homogène d'hydrazinopeptides est obtenu par dilution individuelle de chaque ingrédient dans l'eau ou l'acide acétique dilué, mélange des solutions puis lyophilisation. Cette synthèse est réalisée à partir du mélange des hydrazinopeptides avec le glycomimétique branché à quatre valences [(Qui)₄AKHdz)]. 12 mg d'un mélange équimassique de chacun des 3 hydrazinopeptides (vus en 3)), correspondant à 2,9 micromoles d'hydrazinopeptides sont ajoutés à 3,3 mg (soit 2,2 µmoles) du glycomimétique hydrazine. Au gel formé après humidification par 200 µL d'eau est ajouté le vecteur lipophile (II) (3360 microgrammes, soit 1,14 équivalent peptide) mis en solution dans le 2-méthyl-propan-2-ol (510 µl), sous agitation magnétique avec quelques billes de verre de 5 mm de diamètre. Le volume réactionnel final est de 3.8 ml.

La disparition des hydrazinopeptides au cours du temps est suivie parallèlement à l'apparition des lipopeptides correspondants.

Après 4 heures, le mélange est dilué dans l'eau, congelé et lyophilisé. Les lipopeptides obtenus se présentent sous forme de poudre blanche.

Pour les lipopeptides CChol (avec ou sans glycomimétique) selon 3) et 3'), une étape supplémentaire de purification est nécessaire afin d'éliminer une quantité non négligeable de précurseurs hydrazine n'ayant pas réagis.

Cette purification du milieu réactionnel est réalisée par RP-HPLC sur un système Shimadzu 6A, avec une colonne C4 Deltapak WATERS 15µM 300 Å, 8 mm de diamètre et longueur 100 mm.
Les solvants d'élution (solvants A et B) sont les suivants :
- solvant A : eau désionisée comprenant 0,05% de TFA,
- solvant B (purification) : 0,05% de TFA dans un mélange propan-2-ol / H₂O (40:60).

Le débit d'élution est de 2ml/min en utilisant un gradient linéaire de 0 à 80 % de tampon B en 12 minutes, puis 100 % de tampon B. La fraction de lipopeptide éluée est congelée et lyophilisée.

### 4) et 4') Préparation des mélanges de lipopeptides

Après échange d'ions comme décrit dans l'exemple 1, les mélanges de lipopeptides lyophilisés obtenus en 3) et 3') sont dissous dans une solution d'acide acétique à 10 % avant d'être soumis à une filtration stérilisante sur membrane 0,22 micron (SLGV, Millipore).

### 5) et 5') Préparation des doses de vaccin

### 5) Formulation mannitol - Tween : [3 lipopeptides, vecteur CChol]

Après l'introduction d'excipients cliniquement acceptables (mannitol / lipopeptides 10 / 1 (w/w) et Polysorbate 80 / lipopeptides 2/7 (w/w)) la solution de lipopetides est diluée dans l'eau dans un flacon stérile avant lyophilisation finale.

### 5') Formulation mannitol - glycomimétique lipidé : [3 lipopeptides + glycomimétique, vecteur CChol]

Après l'introduction d'excipients cliniquement acceptables (mannitol / lipopeptides 10/1 (w/w)) la solution de lipopeptides et glycomimétique lipidé est diluée dans l'eau dans un flacon stérile avant lyophilisation finale.

Les doses unitaires de vaccin préconisées pour un essai clinique de phase I chez l'homme sont de 2,5 mg de cocktail lipopeptidique.

### Exemple 4 : Evaluation chez la souris de la capacité des lipopeptides à potentialité vaccinale à induire une réponse immunitaire contre le virus de l'Hépatite C (VHC) (essais pré-cliniques)

### A/ Réponse lympho-proliférative spécifique

Trois peptides, dérivés de la protéine NS3 du virus VHC-1, fonctionnalisés par des groupes α-hydrazinoacétiques au niveau de leur extrémité N-terminale (voir exemple 2) ou de leur extrémité C-terminale (voir protocole de synthèse dans l'exemple 1), ont été lipidés par le vecteur Pam.

L'immunogénicité de ces lipopeptides a été analysée sur des souris syngéniques Balb/c et C57bl/6 après injection sous-cutanée des 3 lipopeptides N-terminal ou C-terminal vecteur Pam (1,2 mg du mélange par souris, équivalent à 100 µg de lipopeptides), soit. 3 peptides natifs (100 µg par souris) + montanide ISA 720, soit 3 peptides natifs seuls (100 µg par souris).

La mesure de la lymphoprolifération est effectuée après la mise en culture des cellules de rate murine, comme indiqué ci-dessous :
Les rates sont récupérées et incubées à 4°C en milieu RPMI 1640 (GIBCO BRL). Les cellules sont ensuite écrasées sur membrane de nylon. Les cellules sont centrifugées à 1500 t/min durant 10 min à 4°C. La lyse des hématies est effectuée en présence de tampon de lyse durant 10 minutes à 4°C. Les cellules sont ensuite centrifugées et reprises en milieu HL-1 (Biowhittaker) contenant 4% de sérum de souris autologues. Les cellules sont ensuite réparties en plaques de cultures 96 puits en polystyrène fond U (Costar) à raison de 100 µl par puits. Les peptides synthétiques natifs (Néosystem) sont dilués en milieu HL-1 (Biowhittaker) ensuite ajoutés à raison de 100 µl par puits pour une concentration finale de 25 µg/ml. Les plaques de culture sont incubées 72 heures à 37°C en atmosphère humide et 5% de CO2. La méthyl-thymidine tritiée (Amersham) est diluée en milieu HL-1 (Amersham) et est ensuite ajoutée dans chaque puits à raison de 1 µCurie dans 25 µl. Après 16 heures d'incubation, les cellules sont collectées sur filtre en fibre de verre (Wallac) avec le système TOMTEC. L'incorporation de thymidine tritiée est ensuite mesurée à l'aide d'un compteur béta (Wallac 140 microbeta).

Les résultats de cette analyse sont exprimés dans le Tableau VIII ci-après.

**Tableau VIIIa et VIIIb :** Mesure de lymphoprolifération (exprimé en index de stimulation)

**Tableau VIIIa : Lipopeptide N terminal**

| | | Immunisation | | | | | |
|---|---|---|---|---|---|---|---|
| | | 3 lipopeptides + vecteur Pam | | 3 peptides natifs /montanide | | 3 peptides natifs seuls | |
| | | Balb/c | C57 | Balb/c | C57 | Balb/c | C57 |
| Antigène (s) de restimulation In vitro | NS3 1007-1037 | 2,31 | 5,052 | 6,54 | 4,75 | 0,599 | 2,43 |
| | NS3 1174-1195 | 1, 406 | 2,02 | 1, 92 | 1, 36 | 0,664 | 1, 38 |
| | NS3 1524-1553 | 6,428 | 2,65 | 14,9 | 1,76 | 0,83 | 2,19 |
| | NS3 1007-1037 + NS3 1174-1195 + NS3 1524-1553 | 6,37 | 6,58 | 3,977 | 5,3 | 1,69 | 3,33 |
| | Contrôle milieu | 1 | 1 | 1 | 1 | 1 | 1 |

**Tableau VIIIb : Lipopeptide C-terminal**

| | | Immunisation | | | | | |
|---|---|---|---|---|---|---|---|
| | | 3 lipopeptides + vecteur Pam | | 3 peptides natifs /montanide | | 3 peptides natifs seuls | |
| | | Balb/c | C57 | Balb/c | C57 | Balb/ c | C57 |
| Antigène (s) De restimulation In vitro | NS3 1007-1037 | 5,09 | 3,66 | 5,27 | 3,57 | 1, 72 | 1, 51 |
| | NS3 1174-1195 | 3,34 | 3,05 | 3,06 | 5, 17 | 2,23 | 2,99 |
| | NS3 1524-1553 | 1,99 | 2,57 | 2,03 | 5,15 | 2,6 | 2,15 |
| | Contrôle milieu | 1 | 1 | 1 | 1 | 1 | 1 |

Les résultats de ces analyses indiquent que les deux types de constructions (position N-terminale et position C-terminale) injectées par voie sous-cutanée à des souris syngéniques Balb/c et C57bl/6 sont capables d'induire une réponse lympho-proliférative spécifique des séquences peptidiques du VHC.

### B/ Réponse humorale

Les 3 lipopeptides en position N-terminale utilisés en A/ ont été testés pour induire une réponse humorale chez la souris. Les 3 peptides natifs et du montanide ISA 720 ont également été testés en témoin.

Les souris ont été immunisées par administration sous-cutanée d'1,2 mg (équivalent à 100 µg de lipopeptides vecteur Pam + mannitol + Tween ou 100 µg d'un mélange de peptides natifs en présence de montanide). Une immunisation suivie d'un rappel à J15 et à J29 ont été réalisé. Les séra ont été prélevés à J14, J28 et J42 après la première administration des antigènes

Le dosage des immunoglobulines spécifiques d'antigènes a été effectué comme suit :

Les antigènes peptidiques sont solubilisés en tampon phosphate 0,1 M pH 8,2 à la concentration de 10 µg/ml. 100 µl de cette solution sont ajoutés par puits, dans une plaque 96 puits Maxisorb (NUNC). Après un nuit d'incubation à 4°C et deux lavages en tampon PBS-tween 0,01%, les puits sont saturés en présence d'un tampon PBS contenant 3% d'albumine bovine sérique (BSA). Après 2 heures d'incubation à température ambiante, 2 lavages des puits sont réalisés en tampon PBS-tween. 100 µl de sérum, dilué au 1/100, sont ensuite ajoutés en incubés une nuit à 4°C. 3 lavages en PBS-tween sont ensuite effectués. L'anticorps secondaire marqué à la péroxidase (anti-IgG (H+L) de souris, Sanofi Pasteur) sont incubés en tampon PBS-BSA 1% à raison de 100 µl par puits durant une heure à température ambiante. Après 4 lavages en PBS-tween, la révélation est effectuée en présence d'orthophényldiamine dihydrochloride (OPD, SIGMA) dilué en tampon de révélation (tampon phosphate-citrate avec perborate de sodium, SIGMA). Après 30 minutes d'incubation à l'abri de la lumière, la réaction est stoppée en présence d'HCL 1M à raison de 50 µl par puits. La lecture est réalisée à une longueur d'onde de 492 nm.

Les résultats sont représentés dans le Tableau IX ci-après.

**Tableau IX : Dosage des anticorps totaux sur souris Balb/c immunisées (résultats exprimés en densité optique).**

| | | Immunisation 3 peptides natifs /montanide | | | Immunisation 3 lipopeptides, vecteur Pam | | |
|---|---|---|---|---|---|---|---|
| Antigène Coaté Sur plaque | NS3 1007-1037 | 0,097 | 0,057 | 0,097 | 0,059 | 0,051 | 0,06 |
| | | 0,052 | 0,118 | 0,093 | 0,078 | 0,052 | 0,056 |
| | | 0,05 | 0,082 | 0,853 | 0,121 | 0,062 | 0,069 |
| | | 0,067 | 0,392 | 0,144 | 0,05 | 0,055 | 0,058 |
| | NS3 1174- 1195 | | | | | | |
| | | 0,16 | 2,553 | 3,246 | 0,052 | 0,053 | 0,138 |
| | | 0,101 | 3,204 | 3, 135 | 0,053 | 0, 055 | 0,05 |
| | | 0,212 | 2,988 | 2,146 | 0,049 | 0,051 | 0,059 |
| | | 0,349 | 2, 052 | 2,873 | 0,05 | 0,047 | 0, 053 |
| | NS3 1524- 1553 | | | | | | |
| | | 0,881 | 2,901 | 2,901 | 0,052 | 0,056 | 0,091 |
| | | 0,364 | 3,204 | 3,204 | 0,048 | 0,051 | 0,06 |
| | | 0,86 | 3,178 | 3,178 | 0,068 | 0,064 | 0,828 |
| | | 0,159 | 2,841 | 2,841 | 0,055 | 0,053 | 0,047 |
| | Témoin négatif | | | | | | |
| | | 0,05 | 0,05 | 0,047 | 0,059 | 0,047 | 0,051 |
| | | 0,056 | 0,056 | 0,079 | 0,078 | 0,079 | 0,071 |
| | | 0,049 | 0,049 | 0,048 | 0,063 | 0,048 | 0,014 |
| | | 0,051 | 0,051 | 0,047 | 0,069 | 0,047 | 0,069 |

Les résultats montrent que les lipopeptides utilisés sont incapables d'induire une réponse humorale caractérisée par la production d'anticorps spécifiques chez ces mêmes modèles murins consécutivement à une immunisation suivie de deux rappels. Ce résultat est d'autant plus original que ces mêmes séquences, lorsqu'elles sont administrées en présence d'un adjuvant huileux comme le Montanide ISA 720n induisent une réponse anticorps très importante, détectable dès la première immunisation. Ce résultat indique que les lipopeptides orientent très fortement l'immunité vers une réponse de type cellulaire.

### C/ Comparaison des vecteurs Pam et CChol

L'immunogénicité de mélanges de lipopeptides a été évaluée chez la souris. Les différentes formulations indiquées dans les exemples 2 et 3 précédant (3 lipopeptides, vecteur Pam; 3 lipopeptides + glycomimétique, vecteur Pam; 3lipopeptides, vecteur CChol ; 3 lipopeptides + glycomimétique, vecteur CChol) ont été testées.

### 1) Protocole d'immunisation sublinguale

Les souris sont immunisées par voie sublinguale avec 480 µg de chaque mélange lipopeptidique dissous dans 5 µl d'eau pour préparation injectable (H₂O ppi). Un rappel est effectué 14 jours après la première immunisation dans les mêmes conditions que précédemment. 14 jours après, les animaux sont sacrifiés, les rates sont prélevées et les cellules spléniques sont mises en culture en présence des différents peptides natifs, seuls ou en mélange, à la concentration de 10 µg/ml

### 2) Dosage de l'Interleukine-2 (IL-2) produite dans les surnageants de culture.

Les anticorps primaires spécifiques de l'IL-2 murine (rat anti-mouse IL-2, Pharmingen 18161D) sont incubés en tampon phosphate 0,1M, pH 8,2 à la concentration de 2 µg/ml. 50 µl par puits de cette solution sont ajoutés dans une plaque 96 puits Maxisorb (NUNC). Après un nuit d'incubation à 4°C et deux lavages en tampon PBS-Tween 0,01%, les puits sont saturés en présence d'un tampon PBS contenant 3% d'albumine bovine sérique (BSA). Après 2 heures d'incubation à température ambiante, 2 lavages sont réalisés en tampon PBS-Tween. 100 µl de surnageant de culture sont ensuite ajoutés et incubés une nuit à 4°C. Une gamme de dilution d'IL-2 murine recombinante est préparée en PBS-BSA 1% (de 20 ng à 0,3 pg) et 100µl de cette préparation sont déposés par puits. 3 lavages en PBS-Tween sont ensuite effectués.

L'anticorps secondaire biotinylé spécifique de l'IL-2 murines (biotin rat anti-mouse IL-2, Pharmingen 18172D) est incubé en tampon PBS-BSA 1% à raison de 100 µl par puits durant une heure à température ambiante. Après 3 lavages en PBS-Tween, la streptavidine-péroxidase (Jackson Immunoresearch), diluée en PBS-Tween à 1mg/ml, est ajoutée à chaque puits dans un volume de 100 µl, durant 30 minutes à température ambiante. Après 4 lavages en PBS-Tween, la révélation est effectuée en présence d'orthophényldiamine dihydrochloride (OPD, SIGMA) dilué en tampon de révélation (tampon phosphate-citrate avec perborate de sodium, SIGMA). Après 30 minutes d'incubation à l'abri de la lumière, la réaction est stoppée en présence d'HCL 1M à raison de 50µl par puits. La lecture est réalisée à une longueur d'onde de 492 nm.

### 3) Autre protocole d'immunisation sublinguale

Les souris (Balb/c) ont été immunisées par administration sublinguale de 5 µl de mélange de lipopeptides (480 µg de poudre équivalent à 40 µg environ de lipopeptides). Un rappel a été effectué 14 jours après cette première immunisation, avec les mêmes quantités de mélange de lipopeptides. Des lots de 5 souris Balb/c (IFFA-CREDO) ont été constitués pour chacune des quatre formulations : [3 lipopeptides + glycomimétique, vecteur CChol] ; [3 lipopeptides, vecteur CChol] ; [3 lipopeptides + glycomimétique, vecteur Pam] ; [3 lipopeptides + glycomimétique, vecteur Pam].

Les souris sont sacrifiées 7 jours après le rappel, les rates sont prélevées et regroupées par lot d'animaux. Les cellules de chaque lot de souris sont restimulées par les différents peptides natifs, soit individuellement soit en mélange (concentration : 10µg/ml). 24 heures après la mise en culture des cellules, les surnageants sont prélevés pour effectuer le dosage de leur contenu en IL-2. Les résultats sont exprimés en picogrammes d'IL-2 par ml de surnageant.

Le Tableau X ci-après présente les résultats d'analyse de l'immunogénicité des différents mélanges lipopeptidiques chez la souris.

**Tableau X : Production d'IL2 après immunisation sublinguale (exprimée en pg/ml).**

| | | Immunisation sublinguale | | | |
|---|---|---|---|---|---|
| | | 3 lipopeptides + vecteur CChol | 3 lipopeptides + glycomimétiques + vecteur CChol | 3 lipopeptides + vecteur Pam | 3 lipopeptides + glycomimétiques + vecteur Pam |
| Antigène(s) De restimulation In vitro | NS3 1007-1037 | 84,38 | 75,22 | 39,21 | 51,33 |
| | NS3 1174-1195 | 28,02 | 28,32 | 0 | 0 |
| | NS3 1524-1553 | 98,34 | 56,65 | 0 | 34,65 |
| | NS3 1007-1037 + NS3 1174-1195 NS3 1524-1553 | 157,97 | 167,79 | 54,36 | 87,17 |
| | Contrôle milieu | 21,9 | 2 | 2 | 2 |

Les résultats obtenus révèlent que les lipopeptides, résultant de la lipidation par le vecteur CChol, induisent une meilleure réponse que les lipopeptides résultant de la lipidation par le vecteur Pam.

Ces lipopeptides CChol s'avère particulièrement immunogènes quand ils sont administrés par voie muqueuse et plus particulièrement par la voie sublinguale.

### Exemple 5 : Evaluation chez le Macaque de la capacité de vaccins lipopeptidiques à induire une réponse de type CTL contre le virus de l'immunodéficience simienne (VIS).

### 1) Synthèse des hydrazinopeptides

L'exemple 1 de la demande de brevet PCT/FR01/02787 décrit la synthèse de peptides fonctionnalisés par des groupements α-hydrazinoacétiques, à partir de séquences peptidiques dérivées des protéines Nef et Gag du virus de l'Immunodéficience Simienne (SIV).

Les séquences peptidiques utilisées ici sont les mêmes que celles décrites dans la demande de brevet PCT/FR01/02787, à l'exception de la séquence SIV1 (voir nouvelle séquence dans le Tableau XI ci-après).

**Tableau XI :**

| Peptides natifs | Hydrazinopeptides |
|---|---|
| Nef 101-126 | VIS 1 HzAc : H-SVRPKVPLRTMSYKLAIDMSHFIKEKK-(COCH₂NHNH₂)-NH₂ |
| Nef 125-147 | VIS 2 HzAc : H-EKGGLEGIYYSARRHRILDMYLEK-(COCH₂NHNH₂)-NH₂ |
| Nef 155-178 | VIS 3 HzAc : H-DWQDYTSGPGIRYPKTFGWLWKLVK-(COCH₂NHNH₂)-NH₂ |
| Nef 201-225 | VIS 4 HzAc : H-SKWDDPWGEVLAWKFDPTLAYTYEAK-(COCH₂NHNH₂)-NH₂ |
| Nef 221-247 | VIS 5 HzAc : H-YTYEAYARYPEELEASQACQRKRLEEGK-(COCH₂NHNH₂)-NH₂ |
| Gag 165-195 | VIS 6 HzAc : H-KFGAEVVPGFQALSEGCTPYDINQMLNCVGDK(COCH₂NHNH₂)-NH₂ |
| Gag 246-282 | VIS 7 HzAc : H-QIQWMYRQQNPIPVGNIYRRWIQLGLQKCVRMYNPTNK-( COCH₂NHNH₂)-NH₂ |

| | |
|---|---|
| * *Comparativement à la séquence VIS 1 décrite dans la demande de brevet N°* PCT/FR01/02787*, la séquence VIS 1' présentée ici comporte deux mutations en position 110 et 112)* | |

### 2) Synthèse du vecteur lipophile

Le vecteur lipophile utilisé est le vecteur IIIa décrit dans l'exemple 1.

### 3) Synthèse des lipopeptides

L'exemple 8 de la demande de brevet PCT/FR01/02787 décrit la méthode de couplage entre les hydrazinopeptides et le vecteur lipophile.

Les appellations et les poids moléculaires des lipopeptides ainsi synthétisés sont donnés dans le Tableau XII ci-après :

**Tableau XII :**

| Lipopeptides | Poids Moléculaire |
|---|---|
| VIS 1 HzPam | 3623.9 |
| VIS 2 HzPam | 3319.35 |
| VIS 3 HzPam | 3463.49 |
| VIS 4 HzPam | 3538.465 |
| VIS 5 HzPam | 3803.716 |
| VIS 6 HzPam | 3852.9 |
| VIS 7 HzPam | 5156.5 |

### 4) Préparation des mélanges de lipopetides

Après échange d'ions comme décrit dans l'exemple 1 par RP-HPLC, en utilisant un gradient court, les mélanges de lipopeptides lyophilisés obtenus en 3) sont dissous dans une solution d'acide acétique à 5 % avant d'être soumis à une filtration stérilisante sur membrane 0,22 micron (SLGV, Millipore).

### 5) Préparation des doses de vaccin

### Formulation mannitol - Tween :

Après l'étape 4), du mannitol a été introduit ainsi que du Tween 80 en quantité suffisante pour la préparation de 10 doses unitaires dont la composition par dose est la suivante :
- 1 Dose représentant 3,5 mg équivalent peptide (0,5 mg par peptide), soit environ 4,2 mg de lipopeptide
- Mannitol apyrogène : environ 42 mg par dose.
- Polysorbate 80 : environ 1 mg par dose.
- Quantité totale de poudre par dose : environ 47 mg
- Conserver à -20°C

La remise en solution des doses obtenues selon cette formulation fournit une solution légèrement opalescente.

### Formulation mannitol-glycomimétique lipidé

Le mélange a été préparé par lapidation simultanée (comme dans l'exemple 3, partie 3')) d'un mélange d'hydrazinopeptides (trifluoroacétate) (5,8 mg par hydrazinopeptide) auquel est ajouté un glycomimétique branché ([(Qui)₄AKHdz)], 12,9 mg).

Après lipidation, le mélange a été dilué puis lyophilisé. Le contre-ion trifluroracétate a été remplacé par un contre-ion acétate par RP-HPLC, en utilisant un gradient court.

Après lyophilisation, la poudre est dissoute dans de l'eau et de l'acide acétique 5% ; avant de soumettre la préparation à la filtration stérilisante sur membrane 0,22 micron SLGV Millipore. Du mannitol a été introduit en quantité suffisante pour la préparation de 11 doses unitaires dont la composition par dose est la suivante :
- 1 Dose représentant 3,4 mg équivalent peptide + glycomimétique (0,5 mg par peptide) soit environ 4,2 mg de lipopeptide
- Mannitol apyrogène : environ 42 mg par dose.
- Quantité totale de poudre par dose : environ 47 mg
- Conserver à -20°C

La remise en solution des doses obtenues selon celle formulation fournit une solution légèrement opalescente.

### 7) Etudes chez le Macaque de la Formulation mannitol-Tween :

Une dose unitaire est utilisée par animal et par injection. La dose est diluée dans de l'eau pour préparation injectable (1 ml par dose) : on obtient une solution opalescente.

Le produit (sans adjuvant) a été administré par voie intra-dermique en 10 points d'injection (100µl par point) (4 macaques), par voie intra-musculaire (4 macaques). Les 3 injections ont été effectuées à 3 semaines d'intervalle et un rappel a été pratiqué 2 mois après la dernière injection. La réponse immune a été testée sur des prélèvements de sang effectués en pré-immunisation, 15 jours après la 3^{ème} immunisation et 14 jours après le rappel.

L'efficacité de l'immunisation a été testée par évaluation du nombre d'effecteurs circulants et de leurs précurseurs sécrétant de l'Interféron gamma (IFN-γ) par un test ELISPOT à la suite d'une restimulation préalable in vitro des cellules mononuclées de sang total (PBMC, Peripheral Blood Mononuclear Cells) par les peptide.

### Préparation des PBMC :

Les PBMC sont isolés par centrifugation en gradient sur un milieu de séparation des lymphocytes (Flow Laboratories, Glasgow, UK ou Pharmacia, Uppsala, Suede) et sont utilisés immédiatement.

### Restimulation in vitro :

Les PBMC (16.10⁶ cellules) sont mises en milieu RPMI 1640 complet à 5 millions/ml ; et aliquotés en 4 tubes de 4 millions de cellules qui seront incubées pendant une nuit à 37°C avec les lipopeptides (10 µg/ml).

Plusieurs lignées sont établies :
- lignée 0 : sans peptides ;
- lignée 1 : avec VIS1 HzPam (LP1) et VIS2 HzPam (LP2) ;
- lignée 2 : avec VIS3 HzPam (LP3), VIS4 HzPam (LP4) et VIS5 HzPam (LP5) ;
- lignée 3 : avec VIS6 HzPam (LP6) et VIS7HzPam (LP7).

Après 1 lavage le Lendemain, les lignées sont placées en plaque 24 puits, à 2 millions de cellules par ml et à raison de 2 ml par puits. Aux jours 3 et 7, de l'Interleukine 2 (IL-2, 10 UI/ mL) est ajoutée avec changement de milieu au jour 7. Ces effecteurs ont été testés après 12 jours de culture et lavés 2 fois juste avant le test.

### Test ELISPOT-IFN-:

Des plaques 96 puits de nitrocellulose (Millipore) sont d'abord sensibilisées une nuit à 4°C avec un anticorps monoclonal de souris anti-IFN-γ simien (clone GZ-4 mabtech) (dilution 1/1000 en tampon phosphate (PBS) : concentration 1 microgramme /ml en final). Après lavages avec du tampon PBS stérile et saturation par du milieu complet pendant 2 heures à 37°C en atmosphère CO₂, les cellules effectrices sont disposées en duplicate (100000 cellules par puits) avec les différents peptides (voir paragraphe suivant), sans IL-2, à la concentration finale de 5 microgrammes par ml, (ou sans peptide pour les contrôles négatifs). L'incubation est maintenue pendant 24 heures à 37°C. Les plaques sont alors lavées plusieurs fois avec du PBS puis du PBS-Tween 0,05 %. Après ces lavages, les plaques sont incubées, durant une nuit à 4°C ou 2 heures à 37°C, avec un anticorps monoclonal de souris anti anti-IFN-γ simien biotinylé dilué à 1/1000 en PBS Tween 0,05% ; BSA 1% (clone 7-B6-1 mabtech, 1 microgramme/ml final).

Après plusieurs lavages la révélation est réalisée au moyen d'Extravidine-phosphatase alcaline (SIGMA) à raison de 2,5 microgramme par ml (incubation 1h à température ambiante). Après des lavages supplémentaires, on développe une coloration mesurable avec un substrat nitro-bleu tétrazolium (BioRad) pendant 15 à 30 minutes. Les plaques sont alors rincées à l'eau et séchées. La lecture du test est réalisée par comptage des spots (centre contrasté et contour diffus) par un stéréomicroscope x 40 (Leica MZ6). Chaque spot violet correspond à une cellule sécrétant de l'IFN-γ, c'est à dire une SFC (Spot Forming Cell).

### Mélanges de petits peptides utilisés pour le test ELISPOT : (Mélanges de 9-15-mers) :

L3.P1 (épitopes de nef 155-178) = 156-165 ; 168-177 ; 165-174
L3.P2 (épitopes de nef 155-178) = 157-165 ; 169-178 ; 166-174
L3.P3 (épitopes de nef 155-178) = 158-167 ; 162-171 ; 167-175
L4.P1 (épitopes de nef 201-225) = 201-211 ; 205-213 ; 211-219 ; 215-225
L4.P2 (épitopes de nef 201-225) = 201-210 ; 205-212 ; 214-223 ; 210-219
L4.P3 (épitopes de nef 201-225) = 203-211 ; 206-215 ; 215-223 ; 204-212
L5.P1 (épitopes de nef 221-247) = 222-229 ; 236-244 ; 225-233
L5.P2 (épitopes de nef 221-247) = 224-233 ; 239-247. Mix6 (épitopes de nef 201-225) = 201-211 ; 205-213 ; 211-219 ; 215-225 ; 201-210 ; 205-212
L7P1 (épitopes de gag 246-281) = 250-257 ; 255-263 ; 261-269 ; 268-276 ; 253-262

### 8) Résultats :

Pour chaque animal sont indiqués dans le Tableau XIII ci-après les mélanges de peptides qui ont permis de détecter des SFC de manière significative 15 jours après la 3^{ème} immunisation ou 14 jours après le rappel :

**TABLEAU XIII**

| | | | | |
|---|---|---|---|---|
| N° SINGE | IMMUNISATION | pré-immun | J15 après Immunisation 3 | J14 après le rappel |
| 1051 | Intra Musculaire | neg | L4P1 | L4P1 |
| 1053 | Intra Dermo | neg | L4P1 | neg |
| 1059 | Intra Dermo | neg | neg | neg |
| 1060 | Intra Dermo | neg | neg | neg |
| 1062 | Intra Musculaire | neg | +/- L4P1 | L4P1 |
| 1066 | Intra Dermo | neg | MIX 6 | neg |
| 1068 | Intra Musculaire | neg | neg | +/- L4P1 |
| 1077 | Intra Musculaire | neg | neg | LP3 ; L4P1 ; L7P1 |

Le 1^{er} groupe (macaques 1053, 1059, 1060, 1066) a reçu le mélange des 7 lipopeptides administré par voie intradermique. Les résultats montrent des réponses positives chez 2 macaques (1053 et 1066) qui reconnaissent 1 pool de petits peptides chacun. Ces réponses sont visibles 15 jours après la dernière immunisation.

Le 2^{ème} groupe (macaques 1051, 1062, 1068, 1077) a reçu le mélange des 7 lipopeptides administré par voie intramusculaire. Trois des 4 macaques ont présenté des réponses CD8 contre 1 pool de petits peptides (2 macaques 1051 et 1062), le 3^{ème} ayant des réponses dirigées contre 3 pools (macaque 1077). Ces réponses étaient visibles 15 jours après la dernière immunisation pour les macaques 1051 et 1062 et 15 jours après le rappel pour les macaques 1051, 1062 et 1077.

Les résultats détaillés obtenus avec la lignée 2 issue de l'animal 1051 sont représentés à la figure 1.

## Revendications

1. Utilisation pour la fabrication d'un vaccin d'un mélange comprenant au moins des lipopeptides, **caractérisée en ce que** lesdits lipopeptides sont constitués d'un composé peptidique lié par un lien hydrazone à au moins un vecteur lipophile de nature non peptidique, et sont de formule générale (I) suivante :
[((R¹)(R²)ᵢ)D-H]j-P (I)
dans laquelle (R¹) (R²)ᵢD représente le vecteur lipophile, dans lequel:
- i représente 0 ou 1,
- dans le cas où i est égal à 0, D représente une liaison,
- dans le cas où i est égal à 1, D représente un hétérocycle saturé, insaturé ou aromatique, mono- ou polycyclique,
- R¹ et R², qui peuvent être identiques ou différents, représentent chacun un groupement de formule L-f-E-f' où L représente un résidu d'un lipide, E représente un bras espaceur, et f et f' représentent des fonctions liant, respectivement, L à E et E à D, et
- P représente le composé peptidique,
- H représente le lien hydrazone formé par la ligation en solution entre une fonction aldéhyde portée par le vecteur lipophile et une fonction dérivé d'hydrazine portée par le composé peptidique,
- j représente 1 à 3.

2. Utilisation selon la revendication 1, **caractérisée en ce que** i est égal à 0, j est égal à 1 et **en ce que** L représente un stérol, un dérivé de stérol ou une chaîne carbonée saturée ou insaturée, linéaire ou ramifiée, comprenant entre 4 et 30 atomes de carbone.

3. Utilisation selon la revendication 2, **caractérisée en ce que** ledit dérivé de stérol est un dérivé du cholestérol et **en ce que** ladite chaîne carbonée répond à la formule

4. Utilisation selon l'une quelconque des revendications 1 à 3, **caractérisée en ce que** E représente une chaîne carbonée linéaire, ramifiée ou cyclique, saturée ou insaturée, comportant de 1 à 18 atomes de carbone et, éventuellement, 1 à 16 hétéroatomes et/ou 1 à 7 groupements sélectionnés parmi les groupements carbonyles, les hétérocycles, les hétéroaryles, les carbocycles et les aryles.

5. Utilisation selon l'une quelconque des revendications 1 à 4, **caractérisée en ce que** f représente une liaison -CO-NH- ou -CO-O- et f' représente une liaison -NH-CO- ou -O-CO-.

6. Utilisation selon la revendication 5, **caractérisée en ce que** le vecteur lipophile répond à la formule (II) :

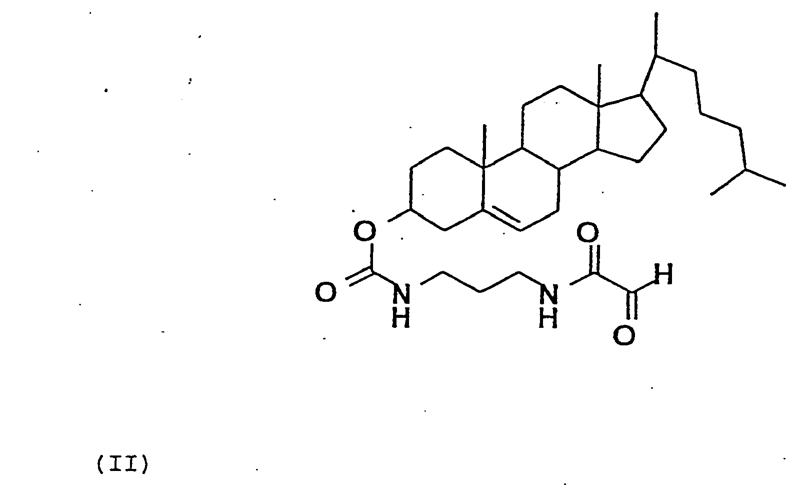

7. Utilisation selon la revendication 5, **caractérisée en ce que** le vecteur lipophile répond à la formule (III) : 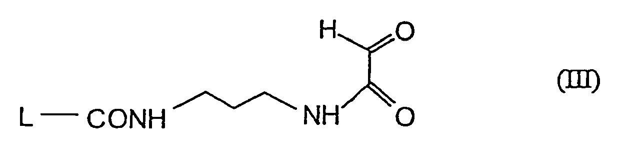 dans laquelle L est tel que défini dans l'une quelconque des revendications 1 à 3.

8. Utilisation selon la revendication 7, **caractérisée en ce que** L représente une chaîne carbonée de formule CH₃-(CH₂)₁₄- dans la formule (III).

9. Utilisation selon l'une des revendications 1 à 8, **caractérisée en ce que** le composé peptidique P est sélectionné dans le groupe constitué par un ou plusieurs antigènes peptidiques et un ou plusieurs glycomimétiques dendrimériques de nature peptidiques.

10. Utilisation selon la revendication 9, **caractérisée en ce que** l'antigène peptidique est choisi parmi le groupe constitué par les peptides et les dérivés de peptides comprenant les glycopeptides et les pseudopeptides.

11. Utilisation selon la revendication 9, **caractérisée en ce que** le glycomimétique dendrimérique de nature peptidique répond à la formule générale (IV) ci-après :
(B²M)ₘ(XN)ₙA (IV)
dans laquelle :
- B² répond à une ou plusieurs formules générales (a) ou (b) ci-après : 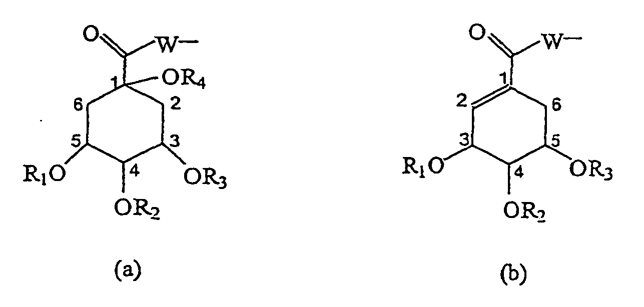 où R₁, R₂, R₃ et R₄ représentent, indépendamment les uns des autres, un atome d'hydrogène ou un groupe protecteur, et où W représente une liaison ou une chaîne carbonée linéaire, ramifiée ou cyclique, saturée ou insaturée, comprenant de 1 à 18 atomes de carbone et éventuellement de 1 à 12 atomes choisis parmi l'oxygène, le soufre et l'azote, ladite chaîne carbonée étant éventuellement substituée par 1 à 16 atomes d'halogène,
- X représente le reste d'un oligopeptide X' comprenant de 1 à 6 acides aminés,
- A représente le reste d'un composé A' au moins trifonctionnel comprenant un enchaînement d'acides aminés, identiques ou différents, sélectionnés dans le groupe constitué par la lysine, l'hydroxylysine, la sérine, la thréonine, la cystéine, l'ornithine, l'acide aspartique et l'acide glutamique
- m est un nombre entier compris entre 1 et 32,
- n est un nombre entier compris entre 0 et 32, et
- M et N représentent chacun un groupe de liaison, respectivement entre B² et A quand n = 0 ou B² et X quand n est différent de 0, et entre X et A quand n est différent de 0, et comprennent, indépendamment l'un de l'autre, une fonction choisie parmi les fonctions oxime, hydrazone, amide, ester, thioester, hydrazide, hydroxamate, éther, thioéther, amine, carbonate, carbamate, thiocarbonate, thiocarbamate, urée, thiourée et thiazolidine.

12. Utilisation selon la revendication 11, **caractérisée en ce que** le composé de formule générale (IV) est tel que m est un nombre entier compris entre 4 et 16, n est un nombre entier compris entre 2 et 8, X représente le reste d'un oligopeptide comprenant de 2 à 4 résidus d'acides aminés, tandis que A représente le reste d'un enchaînement comprenant de 2 à 8 résidus de lysine et se présentant sous la forme d'un dendrimère, et B² représente un reste d'un ou plusieurs composés choisis parmi l'acide (-)-shikimique et l'acide (-)-quinique.

13. Utilisation selon l'une quelconque des revendications 1 à 12, **caractérisée en ce que** le mélange de lipopeptides comprend de 1 à 20 lipopeptides différents, de préférence de 1 à 10 lipopeptides différents.

14. Utilisation selon la revendication 13, **caractérisée en ce que** les lipopeptides sont sous forme de micelles mixtes.

15. Utilisation selon l'une des revendications 1 à 14, **caractérisée en ce que** le composé peptidique est constitué par un antigène dérivé d'un agent infectieux ou d'un cancer.

16. Utilisation selon la revendication 15, **caractérisée en ce que** l'agent infectieux est de type bactérien, parasitaire ou viral.

17. Utilisation selon la revendication 16, **caractérisée en ce que** l'agent infectieux de type viral est le VIH ou le VHC.

18. Utilisation selon la revendication 17,
**caractérisée en ce que** le composé peptidique est une séquence provenant des protéines Gag, Pol ou Nef du virus HIV.

19. Utilisation selon la revendication 18, **caractérisée en ce que** le mélange de lipopeptides contient au moins deux séquences provenant des protéines Gag, Pol et Nef du virus HIV.

20. Utilisation selon la revendication 19, **caractérisée en ce que** le mélange de lipopeptides comprend un mélange de lipopeptides dans lequel les peptides ont les séquences :
- Gag 17-35
- Gag 253-284
- Pol 325-355
- Nef 66-97 et
- Nef 116-147.

21. Utilisation selon l'une quelconque des revendications précédentes, **caractérisée en ce que** le vaccin est formulé pour l'administration par voie transmucosale, transdermique ou par voie intra-musculaire.

22. Utilisation selon la revendication 21, **caractérisée en ce que** le mélange comprend en outre un ou plusieurs excipients cliniquement acceptables.

23. Utilisation selon la revendication 22, **caractérisée en ce que** l'excipient est le mannitol ou le Polysorbate 80 ou un mélange des deux.

24. Utilisation selon la revendication 17, **caractérisée en ce que** les composés peptidiques P de l'agent infectieux VHC sont constitués par les séquences peptidiques suivantes :
- VHC 1 HzAc :
H- K(COCH₂NHNH₂)GREILLGPADGMVSKGWRLLAPITAYAQQTR-NH2
- VHC 2 HzAc :
H-K (COCH₂NHNH₂) GHAVGIFRAAVCTRGVAKAVDF-NH2
- VHC 3 HzAc :
H-K (COCH₂NHNH₂) GAAWYELTPAETTVRLRAYMNTPGLPVAQD-NH2

## Claims

1. Use, for the manufacture of a vaccine, of a mixture including at least lipopeptides, **characterized in that** said lipopeptides are constituted by a peptide compound linked by a hydrazone link to at least one lipophilic vector of a non-peptidic nature, and have the following general formula (I):
[( (R¹) (R²)ᵢ) D-H] j - P (I)
wherein (R¹) (R²)ᵢ D represents the lipophilic vector in which :
- i represents 0 or 1,
- in the case of i being equal to 0, D represents a bond,
- in the case of i being equal to 1, D represents a saturated, unsaturated or aromatic, mono- or polycyclic heterocycle,
- R¹ and R², which can be identical or different, each represent a group having the formula L-f-E-f', where L represents a residue of a lipid, E represents a spacer, and f and f' represent functions linking, respectively, L to E and E to D, and
- P represents the peptide compound,
- H represents the hydrazone link formed by ligation in solution between an aldehyde function carried by the lipophilic vector and a hydrazine derivative function carried by the peptide compound,
- j represents 1 to 3.

2. Use according to claim 1,
**characterized in that** i is equal to 0, j is equal to 1 and **in that** L represents a sterol, a sterol derivative or a saturated or unsaturated, linear or branched carbon chain including between 4 and 30 carbon atoms.

3. Use according to claim 2,
**characterized in that** said sterol derivative is a cholesterol derivative, and **in that** said carbon chain corresponds to the formula CH₃-(CH₂)₁₄-.

4. Use according to any one of claims 1 to 3, **characterized in that** E represents a saturated or unsaturated linear, branched or cyclic carbon chain comprising from 1 to 18 carbon atoms and, as applicable, 1 to 16 heteroatoms and/or 1 to 7 groups selected from the carbonyl groups, heterocycles, heteroaryls, carbocycles and aryls.

5. Use according to any one of claims 1 to 4, **characterized in that** f represents a -CO-NH- or - CO-O- bond and f' represents a -NH-CO- or-O-CO- bond.

6. Use according to claim 5, **characterized in that** the lipophilic vector corresponds to formula (II):

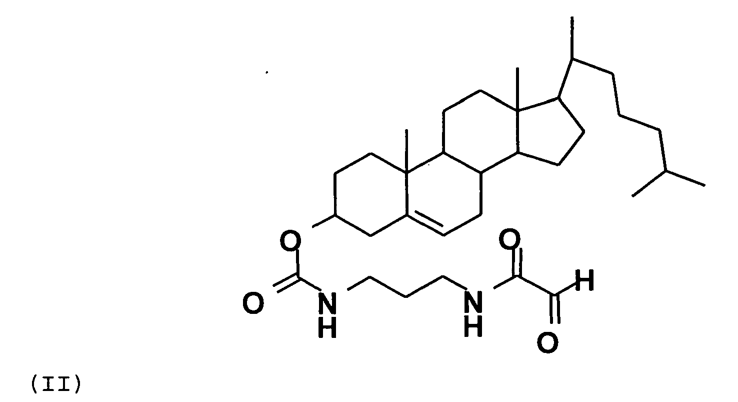

7. Use according to claim 5, **characterized in that** the lipophilic vector corresponds to formula (III): 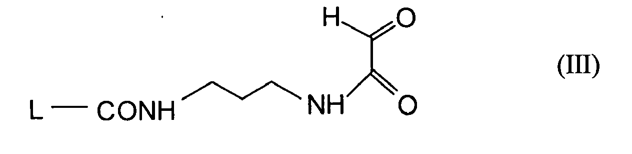 wherein L is as defined in any one of claims 1 to 3.

8. Use according to claim 7, **characterized in that** L represents a carbon chain having the formula CH₃- (CH₂)₁₄- in formula (III).

9. Use according to any one of claims 1 to 8, **characterized in that** the peptide compound P is selected from the group formed by one or more peptidic antigens and one or more dendrimeric glycometics of a peptidic nature.

10. Use according to claim 9, **characterized in that** the peptidic antigen is chosen from the group formed by peptides and peptide derivatives including glycopeptides and pseudopeptides.

11. Use according to claim 9, **characterized in that** the dendrimeric glycomimetic of a peptidic nature corresponds to general formula (IV) bellow :
(B²M)ₘ(XN)ₙA (IV)
wherein :
- B² corresponds to one or more general formulae (a) or (b) below : 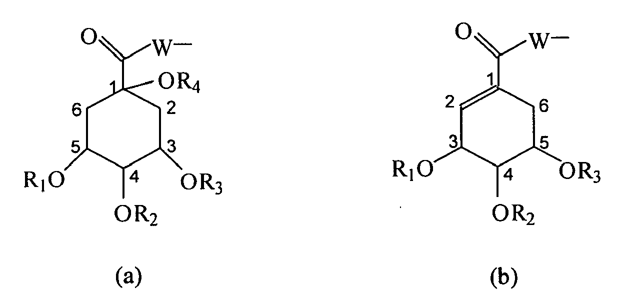 where R₁, R₂, R₃ and R₄ represent, independently of one another, a hydrogen atom or a protective group, and where W represents a bond or a saturated or unsaturated linear, branched or cyclic carbon chain, including from 1 to 18 carbon atoms and possibly from 1 to 12 atoms chosen from oxygen, sulphur and nitrogen, said carbon chain possibly being substituted by 1 to 16 halogen atoms,
- X represents the rest of an X' oligopeptide including from 1 to 6 amino acids,
- A represents the rest of an at least trifunctional A' compound including a chain of identical or different amino acids, selected from the group formed by lysine, hydroxylysine, serine, threonine, cysteine, ornithine, aspartic acid and glutamic acid
- m is an integer between 1 and 32,
- n is an integer between 0 and 32, and
M and N each represent a linking group, respectively, between B² and A when n = 0 or B² and X when n is different from 0, and between X and A when n is different from 0, and include, independently of one another, a function chosen from the oxime, hydrazone, amide, ester, thioester, hydrazide, hydroxamate, ether, thioether, amine, carbonate, carbamate, thiocarbonate, thiocarbamate, urea, thiourea et thiazolidine functions.

12. Use according to claim 11, **characterized in that** the compound having general formula (IV) is such that m is an integer between 4 and 16, n is an integer between 2 and 8, X represents the rest of an oligopeptide including from 2 to 4 amino acids residues, while A represents the rest of a chain including from 2 to 8 lysine residues and taking the form of a dendrimer, and B² represents a rest of one or more components chosen from (-)-shikimic acid and (-)-quinic acid.

13. Use according to any one of claims 1 to 12, **characterized in that** the mixture of lipopeptides includes from 1 to 20 different lipopeptides, preferably from 1 to 10 different lipopeptides.

14. Use according to claim 13, **characterized in that** the lipopeptides are in the form of composite micelles.

15. Use according to any one of claims 1 to 14, **characterized in that** the peptide compound is constituted by an antigen derived from an infectious agent or from a cancer.

16. Use according to claim 15, **characterized in that** the infectious agent is of a bacterial, parasitic or viral type.

17. Use according to claim 16, **characterized in that** the infectious agent of a viral type is the HIV virus or the HCV virus.

18. Use according to claim 17, **characterized in that** the peptide compound is a sequence from the Gag, Pol or Nef proteins of the HIV virus.

19. Use according to claim 18, **characterized in that** the mixture of lipopeptides contains at least two sequences from the Gag, Pol or Nef proteins of the HIV virus.

20. Use according to claim 19, **characterized in that** the mixture of lipopeptides includes a mixture of lipopeptides in which the peptides have the following sequences :
- Gag 17-35
- Gag 253-284
- Pol 325-355
- Nef 66-97 and
- Nef 116-147.

21. Use according to any one of the preceding claims **characterized in that** the vaccine is formulated for transmucosal, transdermal or intramuscular administration.

22. Use according to claim 21, **characterized in that** the mixture further includes one or more clinically acceptable excipients.

23. Use according to claim 22, **characterized in that** the excipient is mannitol or Polysorbate 80, or a mixture of the two.

24. Use according to claim 17, **characterized in that** the peptide compounds P of the infectious agent HCV are constituted by the following peptide sequences :
- HCV 1 HzAc :
H- K (COCH₂NHNH₂) GREILLGPADGMVSKGWRLLAPITAYAQQTR-NH2
- HCV 2 HzAc :
H-K (COCH₂NHNH₂) GHAVGIFRAAVCTRGVAKAVDF-NH2
- HCV 3 HzAc :
H-K(COCH₂NHNH₂)GAAWYELTPAETTVRLRAYMNTPGLPVAQD-NH2

## Patentansprüche

1. Verwendung einer Mischung, die mindestens Lipopeptide umfasst, zur Bereitung eines Impfstoffs, **dadurch gekennzeichnet, dass** die Lipopeptide aus einer Peptidverbindung bestehen, die mit einer Hydrazonbindung an mindestens einen lipophilen Vektor nichtpeptidischer Natur gebunden ist, und die folgende allgemeine Formel (I) aufweisen:
[((R¹)(R²)ᵢ)D-H]j-P (I)
wobei (R¹)(R²)ᵢD für den lipophilen Vektor steht, wobei:
- i für 0 oder 1 steht,
- wenn i gleich 0 ist, D für eine Bindung steht,
- wenn i gleich 1 ist, D für einen gesättigten, ungesättigten oder aromatischen, mono- oder polycyclischen Heterocyclus steht , ,
- R¹ und R², die gleich oder verschieden sein können, jeweils für eine Gruppe der Formel L-f-E-f' stehen, worin L für einen Rest eines Lipids steht, E für einen Spacer-Arm steht, und f und f' für Funktionen stehen, die L mit E bzw. E mit D verbinden, und
- P für die Peptidverbindung steht,
- H für die Hydrazonbindung steht, die gebildet wird durch die Ligation in Lösung zwischen einer Aldehydfunktion, die von dem lipophilen Vektor getragen wird, und einer Funktion, die von dem Hydrazin abgeleitet ist, die von der Peptidverbindung getragen wird,
- j für 1 bis 3 steht.

2. Verwendung nach Anspruch 1, **dadurch gekennzeichnet, dass** i gleich 0 ist, j gleich 1 ist und **dadurch**, dass L für ein Sterol, ein Sterolderivat oder eine gesättigte oder ungesättigte, lineare oder verzweigte Kohlenstoffkette steht, die zwischen 4 und 30 Kohlenstoffatome umfasst.

3. Verwendung nach Anspruch 2, **dadurch gekennzeichnet, dass** das Sterolderivat ein Cholesterolderivat ist, und **dadurch**, dass die Kohlenstoffkette der Formel CH₃-(CH₂)₁₄- entspricht.

4. Verwendung nach irgend einem der Ansprüche 1 bis 3, **dadurch gekennzeichnet, dass** E für eine lineare, verzweigte oder cyclische, gesättigte oder ungesättigte Kohlenstoffkette steht, die 1 bis 18 Kohlenstoffatome und gegebenenfalls 1 bis 16 Heteroatome und/oder 1 bis 7 Gruppen umfasst, ausgewählt aus Carbonyl-, Heterocyclus-, Heteroaryl-, Carbocyclus- und Arylgruppen.

5. Verwendung nach irgendeinem der Ansprüche 1 bis 4, **dadurch gekennzeichnet, dass** f für eine -CO-NH- oder - CO-O-Bindung steht und f' für eine -NH-CO- oder -O-CO-Bindung steht.

6. Verwendung nach Anspruch 5, **dadurch gekennzeichnet, dass** der lipophile Vektor der Formel (II) entspricht:

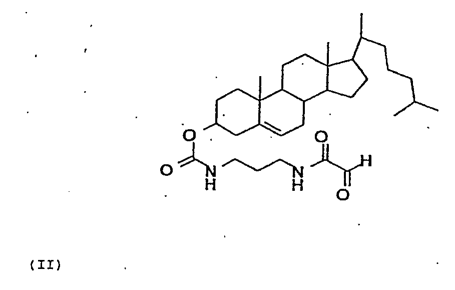

7. Verwendung nach Anspruch 5, **dadurch gekennzeichnet, dass** der lipophile Vektor der Formel (III) entspricht: 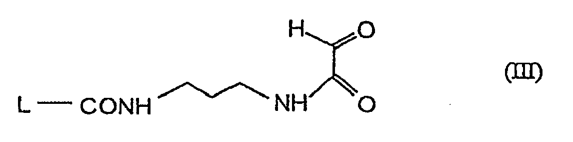 wobei L ist, wie in irgendeinem der Ansprüche 1 bis 3 definiert.

8. Verwendung nach Anspruch 7, **dadurch gekennzeichnet, dass** L für eine Kohlenstoffkette mit der Formel CH₃-(CH₂)₁₄- in Formel (III) steht.

9. Verwendung nach einem der Ansprüche 1 bis 8, **dadurch gekennzeichnet, dass** die Peptidverbindung P ausgewählt ist aus der Gruppe, bestehend aus einem oder mehreren peptidischen Antigenen und einem oder mehreren dendrimeren Glycomimetika petidischer Natur.

10. Verwendung nach Anspruch 9, **dadurch gekennzeichnet, dass** das peptidische Antigen ausgewählt ist aus der Gruppe, bestehend aus Peptiden und Peptidderivaten, die Glycopeptide und Pseudopeptide umfassen.

11. Verwendung nach Anspruch 9, **dadurch gekennzeichnet, dass** das dendrimere Glycomimetikum peptidischer Natur der folgenden allgemeinen Formel (IV) entspricht:
(B²M)ₘ(XN)ₙA (IV)
wobei:
- B² einer oder mehreren der folgenden allgemeinen Formeln (a) oder (b) entspricht: 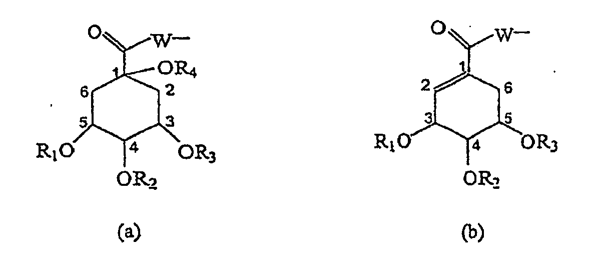 worin R₁, R₂, R₃ und R₄ unabhängig voneinander für ein Wasserstoffatom oder eine Schutzgruppe stehen, und worin W für eine Bindung oder eine lineare, verzweigte oder cyclische, gesättigte oder ungesättigte Kohlenstoffkette steht, die 1 bis 18 Kohlenstoffatome und gegebenenfalls 1 bis 12 Atome, ausgewählt aus Sauerstoff, Schwefel und Stickstoff, umfasst, wobei die Kohlenstoffkette gegebenenfalls durch 1 bis 16 Halogenatome substituiert ist,
- X für den Rest eines Oligopeptids X' steht, der 1 bis 6 Aminosäuren umfasst,
- A für den Rest einer Verbindung A' steht, die mindestens trifunktionell ist, die eine Verkettung aus gleichen oder verschiedenen Aminosäuren, ausgewählt aus der Gruppe, bestehend aus Lysin, Hydroxylysin, Serin, Threonin, Cystein, Ornithin, Asparaginsäure und Glutaminsäure, umfasst,
- m eine ganze Zahl im Bereich zwischen 1 und 32 ist,
- n eine ganze Zahl im Bereich zwischen 0 und 32 ist,
- M und N jeweils für eine Bindungsgruppe zwischen B² und A, wenn n = 0, bzw. B² und X, wenn n nicht 0 ist, bzw. zwischen X und A, wenn n nicht 0 ist, stehen, und unabhängig voneinander eine Funktion, ausgewählt aus Oxim-, Hydrazon-, Amid-, Ester-, Thioester-, Hydrazid-, Hydroxamat-, Ether-, Thioether-, Amin-, Carbonat-, Carbamat-, Thiocarbonat-, Thiocarbamat-, Harnstoff-, Thioharnstoff- und Thiazolidinfunktionen, umfassen.

12. Verwendung nach Anspruch 11, **dadurch gekennzeichnet, dass** die Verbindung der allgemeinen Formel (IV) so ist, dass m eine ganze Zahl im Bereich zwischen 4 und 16 ist, n eine ganze Zahl im Bereich zwischen 2 und 8 ist, X für den Rest eines Oligopeptids steht, der 2 bis 4 Aminosäurereste umfasst, während A für den Rest einer Verkettung steht, die 2 bis 8 Lysinreste umfasst, und die Form eines Dendrimers aufweist, und B² für einen Rest einer oder mehrerer Verbindungen, ausgewählt aus (-)-Shikimisäure und (-)-Chinasäure steht.

13. Verwendung nach irgendeinem der Ansprüche 1 bis 12, **dadurch gekennzeichnet, dass** die Mischung von Lipopeptiden 1 bis 20 verschiedene Lipopeptide, bevorzugt 1 bis 10 verschiedene Lipopeptide umfasst.

14. Verwendung nach Anspruch 13, **dadurch gekennzeichnet, dass** die Lipopeptide die Form gemischter Mizellen aufweisen.

15. Verwendung nach einem der Ansprüche 1 bis 14, **dadurch gekennzeichnet, dass** die Peptidverbindung aus einem Antigen besteht, das von einem Infektionserreger oder einem Krebs abgeleitet ist.

16. Verwendung nach Anspruch 15, **dadurch gekennzeichnet, dass** der Infektionserreger bakterieller, parasitärer oder viraler Art ist.

17. Verwendung nach Anspruch 16, **dadurch gekennzeichnet, dass** der Infektionserreger viraler Art HIV oder HCV ist.

18. Verwendung nach Anspruch 17, **dadurch gekennzeichnet, dass** die Peptidverbindung eine Sequenz ist die aus den Proteinen Gag, Pol oder Nef des HIV-Virus stammt.

19. Verwendung nach Anspruch 18, **dadurch gekennzeichnet, dass** die Mischung von Lipopeptiden mindestens zwei Sequenzen enthält, die aus den Proteinen Gag, Pol und Nef des HIV-Virus stammen.

20. Verwendung nach Anspruch 19, **dadurch gekennzeichnet, dass** die Mischung von Lipopeptiden eine Mischung von Lipopeptiden umfasst, deren Peptide die Sequenzen:
- Gag 17-35
- Gag 253-284
- Pol 325-355
- Nef 66-97 und
- Nef 116-147 aufweisen.

21. Verwendung nach irgendeinem der vorhergehenden Ansprüche, **dadurch gekennzeichnet, dass** der Impfstoff zur transmucosalen, transdermalen oder intramuskulären Verabreichung formuliert ist.

22. Verwendung nach Anspruch 21, **dadurch gekennzeichnet, dass** die Mischung ferner einen oder mehrere klinisch annehmbare Exzipienten umfasst.

23. Verwendung nach Anspruch 22, **dadurch gekennzeichnet, dass** der Exzipient Mannitol oder Polysorbat 80 oder eine Mischung aus beiden ist.

24. Verwendung nach Anspruch 17, **dadurch gekennzeichnet, dass** die Peptidverbindungen P des HCV-Infektionserregers aus den folgenden Peptidsequenzen bestehen:
- HCV 1 HzAc:
H-K(COCH₂NHNH₂) GREILLGPADGMVSKGWRLLAPITAYAQQTR-NH2 - HCV 2 HzAc:
H-K(COCH₂NHNH₂) GHAVGIFRAAVCTRGVAKAVDF-NH2 - HCV 3 HzAc:
H-K(COCH₂NHNH₂) GAAWYELTPAETTVRLRAYMNTPGLPVAQD-NH2


### LISTE DE SEQUENCES

<110> INSTITUT PASTEUR DE LILLE
   CENTRE NATIONAL DE LA RECHERCHE SCIENTIFIQUE (CNRS)
   SOCIETE D'ETUDES ET DE DEVELOPPEMENT DES ANTIGENES
   COMBINATOIRES (SEDAC THERAPEUTICS)
   INSTITUT NATIONAL DE LA SANTE ET DE LA RECHERCHE MEDICALE
   (INSERM)
   UNIVERSITE DE LILLE II
<120> UTILISATION DE MELANGES DE LIPOPEPTIDES POUR LA FABRICATION DE VACCINS
<130> D21144
<140> PCT/FR 03/00 821
   - <141> 2003-03-14
<150> FR 02/03 189
   <151> 2002-03-14
<160> 18
<170> PatentIn version 3.2
<210> 1
   <211> 20
   <212> PRT
   <213> Séquence artificielle
<220>
   <223> Antigène peptidique VIH 1 HzAc, dérivé de la protéine Gag
<220>
   <221> LIPID
   <222> (20)..(20)
   <223> Moitié palmitoyl attachée à l'acide aminé via un espaceur contenant une liaison hydrazone
<220>
   <221> MOD_RES
   <222> (20) .. (20)
   <223> AMIDATION
<400> 1
   Glu Lys Ile Arg Leu Arg Pro Gly Gly Lys Lys Lys Tyr Lys Leu Lys
   1 5 10 15
   His Ile Val Lys
   20
<210> 2
   <211> 33
   <212> PRT
   <213> Séquence artificielle
<220>
   <223> Antigène peptidique VIH 2 HzAc, dérivé de la protéine Gag
<220>
   <221> LIPID
   <222> (33)..(33)
   <223> Moitié palmitoyl attachée à l'acide aminé via un espaceur contenant une liaison hydrazone
<220>
   <221> MOD RES
   <222> (33) .. (33)
   <223> AMIDATION
<400> 2
   Asn Pro Pro Ile Pro Val Gly Glu Ile Tyr Lys Arg Trp Ile Ile Leu
   1 5 10 15
   Gly Leu Asn Lys Ile Val Arg Met Tyr Ser Pro Thr Ser Ile Leu Asp
   20 25 30
   Lys
<210> 3
   <211> 32
   <212> PRT
   <213> Séquence artificielle
<220>
   <223> Antigène peptidique VIH 3 HzAc, dérivé de la protéine Pol
<220>
   <221> LIPID
   <222> (32) .. (32)
   <223> Moitié palmitoyl attachée à l'acide aminé via un espaceur contenant une liaison hydrazone
<220>
   <221> MOD RES
   <222> (32) .. (32)
   <223> AMIDATION
<400> 3
   Ala Ile Phe Gln Ser Ser Met Thr Lys Ile Leu Glu Pro Phe Arg Lys
   1 5 10 15
   Gln Asn Pro Asp Ile Val Ile Tyr Gln Tyr Met Asp Asp Leu Tyr Lys
   20 25 30
<210> 4
   <211> 33
   <212> PRT
   <213> Séquence artificielle
<220>
   <223> Antigène peptidique VIH 4 HzAc, dérivé de la protéine Nef
<220>
   <221> LIPID
   <222> (33) .. (33)
   <223> Moitié palmitoyl attachée à l'acide aminé via un espaceur contenant une liaison hydrazone
<220>
   <221> MOD_RES
   <222> (33) .. (33)
   <223> AMIDATION
<400> 4
   Val Gly Phe Pro Val Thr Pro Gln Val Pro Leu Arg Pro Met Thr Tyr
   1 5 10 15
   Lys Ala Ala Val Asp Leu Ser His Phe Leu Lys Glu Lys Gly Gly Leu
   20 25 30
   Lys
<210> 5
   <211> 33
   <212> PRT
   <213> Séquence artificielle
<220>
   <223> Antigène peptidique VIH 5 HzAc, dérivé de la protéine Nef
<220>
   <221> LIPID
   <222> (33) .. (33)
   <223> Moitié palmitoyl attachée à l'acide aminé via un espaceur contenant une liaison hydrazone
<220>
   <221> MOD_RES
   <222> (33) .. (33)
   <223> AMIDATION
<400> 5
   His Thr Gln Gly Tyr Phe Pro Asp Trp Gln Asn Tyr Thr Pro Gly Pro
   1 5 10 15
   Gly Val Arg Tyr Pro Leu Thr Phe Gly Trp Leu Tyr Lys Leu Val Pro
   20 25 30
   Lys
<210> 6
   <211> 32
   <212> PRT
   <213> Séquence artificielle
<220>
   <223> Antigène peptidique, dérivé de la protéine NS3
<220>
   <221> MOD_RES
   <222> (32)..(32)
   <223> AMIDATION
<400> 6
   Lys Gly Arg Glu Ile Leu Leu Gly Pro Ala Asp Gly Met Val Ser Lys
   1 5 10 15
   Gly Trp Arg Leu Leu Ala Pro Ile Thr Ala Tyr Ala Gln Gln Thr Arg
   20 25 30
<210> 7
   <211> 32
   <212> PRT
   <213> Séquence artificielle
<220>
   <223> Antigène peptidique VHC 1 HzAc, dérivé de la protéine NS3
<220>
   <221> LIPID
   <222> (1) .. (1)
   <223> Moitié palmitoyl attachée à l'acide aminé via un espaceur contenant une liaison hydrazone
<220>
   <221> MOD_RES
   <222> (32)..(32)
   <223> AMIDATION
<400> 7
   Lys Gly Arg Glu Ile Leu Leu Gly Pro Ala Asp Gly Met Val Ser Lys
   1 5 10 15
   Gly Trp Arg Leu Leu Ala Pro Ile Thr Ala Tyr Ala Gln Gln Thr Arg
   20 25 30
<210> 8
   <211> 23
   <212> PRT
   <213> Séquence artificielle
<220>
   <223> Antigène peptidique, dérivé de la protéine NS3
<220>
   <221> MOD_RES
   <222> (23)..(23)
   <223> AMIDATION
<400> 8
   Lys Gly His Ala Val Gly Ile Phe Arg Ala Ala Val Cys Thr Arg Gly
   1 5 10 15
   Val Ala Lys Ala Val Asp Phe
   20
<210> 9
   <211> 23
   <212> PRT
   <213> Séquence artificielle
<220>
   <223> Antigène peptidique VHC 2 HzAc, dérivé de la protéine NS3
<220>
   <221> LIPID
   <222> (1) .. (1)
   <223> Moitié palmitoyl attachée à l'acide aminé via un espaceur contenant une liaison hydrazone
<220>
   <221> MOD RES
   <222> (23)..(23) <223> AMIDATION
<400> 9
   Lys Gly His Ala Val Gly Ile Phe Arg Ala Ala Val Cys Thr Arg Gly
   1 5 10 15
   Val Ala Lys Ala Val Asp Phe
   20
<210> 10
   <211> 31
   <212> PRT
   <213> Séquence artificielle
<220>
   <223> Antigène peptidique, dérivé de la protéine NS3
<220>
   <221> MOD_RES
   <222> (31) .. (31) <223> AMIDATION
<400> 10
   Lys Gly Ala Ala Trp Tyr Glu Leu Thr Pro Ala Glu Thr Thr Val Arg
   1 5 10 15
   Leu Arg Ala Tyr Met Asn Thr Pro Gly Leu Pro Val Ala Gln Asp
   20 25 30
<210> 11
   <211> 31
   <212> PRT
   <213> Séquence artificielle
<220>
   <223> Antigène peptidique VHC 3 HzAc, dérivé de la protéine NS3
<220>
   <221> LIPID
   <222> (1)..(1)
   <223> Moitié palmitoyl attachée à l'acide aminé via un espaceur contenant une liaison hydrazone
<220>
   <221> MOD_RES
   <222> (31) .. (31)
   <223> AMIDATION
<400> 11
   Lys Gly Ala Ala Trp Tyr Glu Leu Thr Pro Ala Glu Thr Thr Val Arg
   1 5 10 15
   Leu Arg Ala Tyr Met Asn Thr Pro Gly Leu Pro Val Ala Gln Asp
   20 25 30
<210> 12
   <211> 27
   <212> PRT
   <213> Séquence artificielle
<220>
   <223> Antigène peptidique VIS 1 HzAc, dérivé de la protéine Nef
<220>
   <221> LIPID
   <222> (27)..(27)
   <223> Moitié palmitoyl attachée à l'acide aminé via un espaceur contenant une liaison hydrazone
<220>
   <221> MOD_RES
   <222> (27) .. (27)
   <223> AMIDATION
<400> 12
   Ser Val Arg Pro Lys Val Pro Leu Arg Thr Met Ser Tyr Lys Leu Ala
   1               5                   10                  15
   Ile Asp Met Ser His Phe Ile Lys Glu Lys Lys
               20                  25
<210> 13
   <211> 24
   <212> PRT
   <213> Séquence artificielle
<220>
   <223> Antigène peptidique VIS 2 HzAc, dérivé de la protéine Nef
<220>
   <221> LIPID
   <222> (24)..(24)
   <223> Moitié palmitoyl attachée à l'acide aminé via un espaceur contenant une liaison hydrazone
<220>
   <221> MOD_RES
   <222> (24) .. (24) <223> AMIDATION
<400> 13
   Glu Lys Gly Gly Leu Glu Gly Ile Tyr Tyr Ser Ala Arg Arg His Arg
   1               5                   10                  15
   Ile Leu Asp Met Tyr Leu Glu Lys
               20
<210> 14
   - <211> 25
   <212> PRT
   <213> Séquence artificielle
<220>
   <223> Antigène peptidique VIS 3 HzAc, dérivé de la protéine Nef
<220>
   <221> LIPID
   <222> (25) .. (25)
   <223> Moitié palmitoyl attachée à l'acide aminé via un espaceur contenant une liaison hydrazone
<220>
   <221> MOD_RES
   <222> (25) .. (25) <223> AMIDATION
<400> 14
   Asp Trp Gln Asp Tyr Thr Ser Gly Pro Gly Ile Arg Tyr Pro Lys Thr
   1               5                   10                  15
   Phe Gly Trp Leu Trp Lys Leu Val Lys
               20                  25
<210> 15
   <211> 26
   <212> PRT
   <213> Séquence artificielle
<220>
   <223> Antigène peptidique VIS 4 HzAc, dérivé de la protéine Nef
<220>
   <221> LIPID
   <222> (26)..(26)
   <223> Moitié palmitoyl attachée à l'acide aminé via un espaceur contenant une liaison hydrazone
<220>
   <221> MOD_RES
   <222> (26) .. (26)
   <223> AMIDATION
<400> 15
   Ser Lys Trp Asp Asp Pro Trp Gly Glu Val Leu Ala Trp Lys Phe Asp
   1               5                   10                  15
   Pro Thr Leu Ala Tyr Thr Tyr Glu Ala Lys
               20                  25
<210> 16
   <211> 28
   <212> PRT
   <213> Séquence artificielle
<220>
   <223> Antigène peptidique VIS 5 HzAc, dérivé de la protéine Nef
<220>
   <221> LIPID
   <222> (28)..(28)
   <223> Moitié palmitoyl attachée à l'acide aminé via un espaceur contenant une liaison hydrazone
<220>
   <221> MOD RES
   <222> (28) .. (28)
   <223> ACETYLATION
<400> 16
   Tyr Thr Tyr Glu Ala Tyr Ala Arg Tyr Pro Glu Glu Leu Glu Ala Ser
   1               5                   10                  15
   Gln Ala Cys Gln Arg Lys Arg Leu Glu Glu Gly Lys
               20                  25
<210> 17
   <211> 32
   <212> PRT
   <213> Séquence artificielle
<220>
   <223> Antigène peptidique VIS 6 HzAc, dérivé de la protéine Gag
<220>
   <221> LIPID
   <222> (32) .. (32)
   <223> Moitié palmitoyl attachée à l'acide aminé via un espaceur contenant une liaison hydrazone
<220>
   <221> MOD_RES
   <222> (32)..(32) <223> AMIDATION
<400> 17
   Lys Phe Gly Ala Glu Val Val Pro Gly Phe Gln Ala Leu Ser Glu Gly
   1               5                   10                  15
   Cys Thr Pro Tyr Asp Ile Asn Gln Met Leu Asn Cys Val Gly Asp Lys
               20                  25                  30
<210> 18
   <211> 38
   <212> PRT
   <213> Séquence artificielle
<220>
   <223> Antigène peptidique VIS 7 HzAc, dérivé de la protéine Gag
<220>
   <221> LIPID
   <222> (38) .. (38)
   <223> Moitié palmitoyl attachée à l'acide aminé via un espaceur contenant une liaison hydrazone
<220>
   <221> MOD RES
   <222> (38) .. (38) <223> AMIDATION
<400> 18
   Gln Ile Gln Trp Met Tyr Arg Gln Gln Asn Pro Ile Pro Val Gly Asn
   1               5                   10                  15
   Ile Tyr Arg Arg Trp Ile Gln Leu Gly Leu Gln Lys Cys Val Arg Met
               20                  25                  30
   Tyr Asn Pro Thr Asn Lys
           35

### LISTE DE SEQUENCES

<110> INSTITUT PASTEUR DE LILLE
   CENTRE NATIONAL DE IrA RECHERCHE SCIENTIFIQUE (CNRS)
   SOCIETE D'ETUDES ET DE DEVELOPPEMENT DES ANTIGENES
   COMBINATOIRES (SEDAC THERAPEUTICS)
   INSTITUT NATIONAL DE LA SANTE ET DE LA RECHERCHE MEDICALE
   (INSERM)
   UNIVERSITE DE LILLE II
<120> UTILISATION DE MELANGES DE LIPOPEPTIDES POUR LA FABRICATION DE VACCINS
<130> D21144
<140> PCT/FR 03/00 821 - <141> 2003-03-14
<150> FR 02/03 189 <151> 2002-03-14
<160> 18
<170> PatentIn version 3.2
<210> 1
   <211> 20
   <212> PRT
   <213> Séquence artificielle
<220>
   <223> Antigène peptidique VIH 1 HzAc, dérivé de la protéine Gag
<220>
   <221> LIPID
   <222> (20) .. (20)
   <223> Moitié palmitoyl attachée à l'acide aminé via un espaceur contenant une liaison hydrazone
<220>
   <221> MOD_RES
   <222> (20) .. (20)
   <223> AMIDATION
<400> 1
   Glu Lys Ile Arg Leu Arg Pro Gly Gly Lys Lys Lys Tyr Lys Leu Lys
   1 5 10 15
   His Ile Val Lys
   20
<210> 2
   <211> 33
   <212> PRT
   <213> séquence artificielle
<220>
   <223> Antigène peptidiques VIH 2 HzAc, dérivé de la protéine Gag
<220>
   <221> LIPID
   <222> (33) .. (33)
   <223> Moitié palmitoyl attachée à l'acide aminé via un espaceur, contenant une liaison hydrazone
<220>
   <221> MOD_RES
   <222> (33) .. (33)
   <223> AMIDATION
<400> 2
   Asn Pro Pro Ile Pro Val Gly Glu Ile Tyr Lys Arg Trp Ile Ile Leu
   1 5 10 15
   Gly Leu Asn Lys Ile Val Arg Met Tyr Ser Pro Thr Ser Ile Leu Asp
   20 25 30
   Lys
<210> 3
   <211> 32
   <212> PRT
   <213> Séquence artificielle
<220>
   <223> Antigène peptidique VIH 3 HzAc, dérivé de la protéine Pol
<220>
   <221> LIPID
   <222> (32).. (32)
   <223> Moitié palmitoyl attachée à l'acide aminé via un espaceur contentant une liaison hydrazone
<220>
   <221> MOD_RES
   <222> (32) .. (32)
   <223> AMIDATION
<400> 3
   Ala Ile Phe Gln Ser Ser Met Thr Lys Ile Leu Glu Pro Phe Arg Lys
   1 5 10 15
   Gln Asn Pro Asp Ile Val Ile Tyr Gln Tyr Met Asp Asp Leu Tyr Lys
   20 25 30
<210> 4
   <211> 33
   <212> PRT
   <213> séquence artificielle
<220>
   <223> Antigène peptidique VIH 4 HzAc, dérivé de la protéine Nef
<220>
   <221> LIPID
   <222> (33).. (33)
   <223> Moitié palmitoyl attachée à l'acide aminé via un espaceur contenant une liaison hydrazone
<220>
   <221> MOD_RES
   <222> (33).. (33)
   <223> AMIDATION
<400> 4
   Val Gly Phe Pro Val Thr Pro Gln Val Pro Leu Arg Pro Met Thr Tyr
   1 5 10 15
   Lys Ala Ala Val Asp Leu Ser His Phe Leu Lys Glu Lys Gly Gly Leu
   20 25 30
   Lys
<210> 5
   <211> 33
   <212> PRT
   <213> Séquence artificielle
<220>
   <223> Antigène peptidique VIH 5 HzAc, dérivé de la protéine Nef
<220>
   <221> LIPID
   <222> (33) .. (33)
   <223> Moitié palmitoyl attachée à l'acide aminé via un espaceur contenant une liaison hydrazone
<220>
   <221> MOD_RES
   <222> (33).. (33)
   <223> AMIDATION
<400> 5
   His Thr Gln Gly Tyr Phe Pro Asp Trp Gln Asn Tyr Thr Pro Gly Pro
   1 5 10 15
   Gly Val Arg Tyr Pro Leu Thr Phe Gly Trp Leu Tyr Lys Leu Val Pro
   20 25 30
   Lys
<210> 6
   <211> 32
   <212> PRT
   <213> séquence artificielle
<220>
   <223> Antigène peptidique, dérivé de la protéine NS3
<220>
   <221> MOD_RES
   <222> (32).. (32)
   <223> AMIDATION
<440> 6
<210> 7
   <211> 32
   <212> PRT
   <213> Séquence artificielle
<220>
   <223> Antigène peptidique VHC 1 HzAc, dérivé de la protéine NS3
<220>
   <221> LIPID
   <222> (1)..(1)
   <223> Moitié palmitoyl attachée à l'acide aminé via un espaceur contenant une liaison hydrazone,
<220>
   <221> MOD_RES
   <222> (32) .. (32)
   <223> AMIDATION
<400> 7
   Lys Gly Arg Glu Ile Leu Leu Gly Pro Ala Asp Gly Met Val Ser Lys
   1 5 10 15
   Gly Trp Arg Leu Leu Ala Pro Ile Thr Ala Tyr Ala Gln Gln Thr Arg
   20 25 30
<210> 8
   <211> 23
   <212> PRT
   <213> Séquence artificielle
<220>
   <223> Antigène peptidiques, dérivé de la protéine NS3
<220>
   <221> MOD_RES
   <222> (23) .. (23)
   <223> AMIDATION
<440> 8
<210> 9
   <211> 23
   <212> PRT
   <213> Séquence artificielle
<220>
   <223> Antigène peptidiques VHC 2 MzAc, dérivé de la protéine NS3
<220>
   <221> LIPID
   <222> (1)..(1)
   <223> Moitié palmitoyl attachée à l'acide aminé via un espaceur contenant une liaison hydrazone
<220>
   <221> MOD_RES
   <222> (23)..(23)
   <223> AMIDATION
<400> 9
   Lys Gly His Ala Val Gly Ile Phe Arg Ala Ala Val Cys Thr Arg Gly
   1 5 10 15
   Val Ala Lys Ala Val Asp Phe
   20
<210> 10
   <211> 31
   <212> PRT
   <213> séquence artificielle
<220>
   <223> Antigène peptidique, dérivé de la protéine N3
<220>
   <221> MOD_RES
   <222> (31) .. (31)
   <223> AMIDATION
<400> 10
   Lys Gly Ala Ala Trp Tyr Glu Leu Thr Pro Ala Glu Thr Thr Val Arg
   1 5 10 15
   Leu Arg Ala Tyr Met Asn Thr Pro Gly Leu Pro Val Ala Gln Asp
   20 25 30
<210> 11
   <211> 31
   <212> PRT
   <213> Séquence artificielle
<220>
   <223> Antigène peptidique VHC 3 HzAc, dérivé de la protéines NS3
<220>
   <221> LIPID
   <222> (1) .. (1)
   <223> Moitié palmitoyl attachée à l'acide aminé via un espaceur contenant une liaison hydrazone
<220>
   <221> MOD_RES
   <222> (31) .. (31)
   <223> AMIDATION
<400> 11
   Lys Gly Ala Ala Trp Tyr Glu Leu Thr Pro Ala Glu Thr Thr Val Arg
   1 5 10 15
   Leu Arg Ala Tyr Met Asn Thr Pro Gly Leu Pro Val Ala Gln Asp
   20 25 30
<210> 12
   <211> 27
   <212> PRT
   <213> Séquence artificielle
<220>
   <223> Antigène peptidique VIS 1 HZAC, dérivé de la protéine Nef
<220>
   <221> LIPID
   <222> (27) .. (27)
   <223> Moitié palmitoyl attachée à l'acide aminé via un espaceur, contenant une liaison hydrazone
<220>
   <221> MOD_RES
   <222> (27)..(27)
   <223> AMIDATION
<400> 12
   Ser Val Arg Pro Lys Val Pro Leu Arg Thr Met Ser Tyr Lys Leu Ala
   1               5                   10                  15
   Ile Asp Met Ser His Phe Ile Lys Glu Lys Lys
               20                  25
<210> 13
   <211> 24
   <212> PRT
   <213> séquence artificielle
<220>
   <223> Antigène peptidique VIS 2 HzAc, dérivé de la protéine Nef
<220>
   <221> LIPID
   <222> (24) .. (24)
   <223> Moitié palmitoyl attachée à l'acide aminé via un espaceur contenant une liaison hydrazone
<220>
   <221> MOD_RES
   <222> (24) .. (24)
   <223> AMIDATION
<400> 13
   Glu Lys Gly Gly Leu Glu Gly Ile Tyr Tyr Ser Ala Arg Arg His Arg
   1               5                   10                  15
   Ile Leu Asp Met Tyr Leu Glu Lys
               20
<210> 14
   <211> 25
   <212> PRT
   <213> Séquence artificielle
<220>
   <223> Antigène peptidique VIS 3 HzAc, dérivé de la protéine Nef
<220>
   <221> LIPID
   <222> (25) .. (25)
   <223> Moitié palmitoyl attachée à l'acide aminé via un espaceur contenant une liaison hydrazone
<220>
   <221> MOD_RES
   <222> (25).. (25)
   <223> ANIMATION
<400> 14
   Asp Trp Gln Asp Tyr Thr Ser Gly Pro Gly Ile Arg Tyr Pro Lys Thr
   1               5                   10                  15
   Phe Gly Trp Leu Trp Lys Leu Val Lys
               20                  25
<210> 15
   <211> 26
   <212> PRT
   <213> Séquence artificielle
<220>
   <223> Antigène peptidique VIS 4 HzAc, dérivé de la protéine Nef
<220>
   <221> LIPID
   <222> (26) .. (26)
   <223> Moitié palmitoyl attachée à l'acide aminé via un espaceur contenant une liaison hydrazone
<220>
   <221> MOD_RES
   <222> (26).. (26)
   <223> AMIDATION
<400> 15
   Ser Lys Trp Asp Asp Pro Trp Gly Glu Val Leu Ala Trp Lys Phe Asp
   1               5                   10                  15
   Pro Thr Leu Ala Tyr Thr Tyr Glu Ala Lys
               20                  25
<210> 16
   <211> 26
   <212> PRT
   <213> Séquence artificielle
<220>
   <223> Antigène peptidique VIS 5 HzAc, dérivé de la protéine Nef
<220>
   <221> LIPID
   <222> (28) .. (28)
   <223> Moitié palmitoyl attachée à l'acide aminé via un espaceur contenant une liaison hydrazone
<220>
   <221> MOD_RES
   <222> (28)..(28)
   <223> ACETYLATION
<400> 16
<210> 17
   <211> 32
   <212> PRT
   <213> Séquence artificielle
<220>
   <223> Antigène peptidique VIS 6 HzAc, dérivé de la protéine Gag
<220>
   <221> LIPID
   <222> (32) .. (32)
   <223> Moitié palmitoyl attachée à l'acide aminé via un espaceur contenant une liaison hydrazone
<220>
   <221> MOD_RES
   <222> (32) .. (32)
   <223> AMIDATION
<400> 17
   Lys Phe Gly Ala Glu Val Val Pro Gly Phe Gln Ala Leu Ser Glu Gly
   1 5 10 15
   Cys Thr Pro Tyr Asp Ile Asn Gln Met Leu Asn Cys Val Gly Asp Lys
   20 25 30
<210> 18
   <211> 38
   <212> PRT
   <213> Séquence artificielle
<220>
   <223> Antigène peptidique VIS 7 HzAc, dérivé de la protéine Gag
<220>
   <221> LIPID
   <222> (38).. (38)
   <223> Moitié palmitoyl attachée à l'acide aminé via un espaceur contenant une liaison hydrazone,
<220>
   <221> MOD_RES
   <222> (38) .. (38)
   <223> AMIDATION
<400> 18
   Gln Ile Gln Trp Met Tyr Arg Gln Gln Asn Pro Ile Pro Val Gly Asn
   1               5                   10                  15
   Ile Tyr Arg Arg Trp Ile Gln Leu Gly Leu Gln Lys Cys Val Arg Met
               20                  25                  30
   Tyr Asn Pro Thr Asn Lys
           35